# EUROPEAN PATENT SPECIFICATION

(11) **EP 2 793 927 B1**
(45) Date of publication and mention of the grant of the patent: **06.03.2019**
(21) Application number: 12858768.0
(22) Date of filing: 21.12.2012
(51) Int. Cl.: A61K 38/17, A61K 39/395, C07K 14/47, C07K 16/46, A61P 29/00

(54) **SERUM AMYLOID P-ANTIBODY FUSION PROTEINS**
SERUM-AMYLOID-P-ANTIKÖRPER-FUSIONSPROTEINE
PROTÉINES DE FUSION DE P-ANTICORPS SÉRUM AMYLOÏDE

(30) Priority: 21.12.2011 US 201161578498 P
(43) Date of publication of application: 29.10.2014
(73) Proprietor: Promedior, Inc., Lexington, MA 02421 (US)
(72) Inventor: LUPHER, Mark, L., Jr., Lexington, MA 02421 (US); WILLETT, W., Scott, Doylestown, PA 18902-1593 (US)
(74) Representative: Vossius & Partner Patentanwälte Rechtsanwälte mbB
(86) International application number: PCT/US2012/071394
(87) International publication number: WO 2013/096847

(56) References cited:
- WO-A1-2010/148234
- WO-A2-2009/009019
- US-A- 5 221 628
- BRISTOW C L ET AL: "Evidence for the binding of human serum amyloid P component to Clq and Fabgamma", MOLECULAR IMMUNOLOGY, PERGAMON, GB, vol. 23, no. 10, 1 October 1986 (1986-10-01), pages 1045-1052, XP027201033, ISSN: 0161-5890 [retrieved on 1986-10-01]
- BROWN, M. ET AL.: 'Receptor-Ligand Interactions between Serum Amyloid P Component and Model Soluble Immune Complexes' THE JOURNAL OF IMMUNOLOGY vol. 151, no. 4, 1993, pages 2087 - 2095, XP055076326
- NIELSEN, E. ET AL.: 'Calcium-enhanced aggregation of serum amyloid P component and its inhibition by the ligands heparin and heparan sulphate' ACTA PATHOLOGICA, MICROBIOLOGICA, ET IMMUNOLOGICA SCANDINAVICA vol. 102, 1994, pages 420 - 426, XP009035059
- THIE H ET AL: "Multimerization domains for antibody phage display and antibody production", NEW BIOTECHNOLOGY, ELSEVIER BV, NL , vol. 26, no. 6 1 December 2009 (2009-12-01), pages 314-321, XP002703962, ISSN: 1871-6784, DOI: 10.1016/J.NBT.2009.07.005 Retrieved from the Internet: URL:http://www.sciencedirect.com/science/a rticle/pii/S1871678409011029 [retrieved on 2009-07-22]

## Description

The advent of recombinant DNA technology has provided the possibility of large scale production of biologically active proteins for therapeutic use. Accordingly, there are now many recombinantly produced protein products in the clinic or under development, including large proteins (e.g., erythropoietin), small peptide fragments, and antibodies as well as antigen- binding fragments thereof.

However, there are many common difficulties associated with the production and use of such recombinant proteins in human therapies. For example, many recombinantly produced and purified proteins are characterized by short *in vivo* half-life and/or weak binding affinity for their *in vivo* targets thereby reducing their usefulness as therapeutic agents.

In certain instances, it has been found that the stability and/or biological activity of a protein or peptide fragment thereof can be enhanced by fusing it to another heterologous polypeptide domain (e.g., a stabilization domain such as immunoglobulin Fc domains) or by generating multimeric complexes of the active protein. For example, it has been found that multimerization of proteins is often an effective way of increasing the half-life of these agents thus allowing them to exert their activity over a longer time scale. Furthermore, many biologically active proteins have been found to be more potent *in vivo* when in the form of an oligomeric structure. In many cases, this increased activity is due to factors such as binding with avidity rather than affinity and/or the ability to cross-link molecules (e.g. identical receptor subunits as in the insulin receptor that are activated through dimerization). These properties of increased half-life and avidity enable lower doses of the protein and peptide molecules to be used thereby potentially reducing cost of therapy as well as any dose-dependent side-effects.

Different approaches have been proposed for making multimers of recombinant proteins. For example, chemical linkage of proteins to polymers such as polyethylene glycol has been attempted (Katre et al., (1987) Proc. Natl. Acad. Sci. USA 84, 1487). However, this technique is generally cumbersome and requires large amounts of purified material. In antibody molecules, modifications in the hinge and other regions of the protein have been attempted in order to modulate the extent to which antibodies will associate with each other. Results however have been inconsistent and unpredictable. Similarly, use of protein A fusions to generate multimeric antibodies may successfully link antibody fragments, but is of limited application in other fields.

WO 2010/148234 describes serum amyloid P (SAP) variants and their use. Thie H et al (NEW BIOTECHNOLOGY 26, 314-321 (2009)) describes multimerization domains for antibody phage display and antibody production.

Described herein are novel scaffold molecules for delivering molecular entities effectively as therapeutic, prophylactic, or diagnostic agents.

The invention is defined by the claims. Accordingly, in one aspect, the present invention relates to a fusion protein comprising a serum amyloid P (PTX-2) pentamer having at least one PTX-2 protomer fused with an antibody or antigen-binding antibody fragment; wherein the antibody or antigen-binding antibody fragment is fused to the N-terminus of at least one PTX-protomer; and wherein the fusion protein further comprises a linker between the antibody or antigen-binding antibody fragment and the at least one PTX-2 protomer.

Described is the use of pentraxin-2 (PTX-2) as a scaffold molecule for antibody and antibody fragment (e.g., antigen-binding antibody fragments) based therapeutic agents. One or more antibody and antibody fragment may be linked to a PTX-2 protomer to form a functionalized PTX-2 protomer, and these functionalized PTX-2 protomers may be combined to form functionalized PTX-2 pentamers.

In certain aspects, the disclosure is directed to a composition comprising a PTX-2 pentamer having at least one, at least two, at least three, or at least four PTX-2 protomers with an attached antibody or antibody fragment. In other aspects, the disclosure is directed to compositions wherein the PTX-2 pentamer comprises five protomers with attached antibodies or antibody fragments.

In certain aspects, the PTX-2 pentamers of the disclosure comprise one or more (e.g., one, two, three, or four) unmodified PTX-2 protomers.

In certain aspects, the PTX-2 pentamers of the disclosure comprise one or more (e.g., one, two, three, four, or five) PTX-2 protomers that are modified to eliminate a ligand-binding site on naturally occurring PTX-2 protomers, a Ca⁺² binding site on naturally occurring PTX-2 protomers, or a combination thereof.

In certain aspects, the antibody or antibody fragment are attached to the C-terminus or the N-terminus of at least one, at least two, at least three, at least four, or all five PTX-2 protomers. In other aspects, the antibody or antibody fragments are attached to both the C-terminus and N-terminus of at least one, at least two, at least three, at least four, or all five PTX-2 protomers.

In certain aspects, at least one (e.g., one, two, three, four, or five) PTX-2 protomers of the disclosure may comprise a linker between the antibody or antibody fragment and at least one PTX-2 protomer.

In certain aspects, the antibody or antibody fragment is inserted within at least one (e.g., one, two, three, four, or five) PTX-2 protomer.

In certain aspects, the PTX-2 pentamer comprises one or more (e.g., one, two, three, four, or five) PTX-2 protomers having at least one additional cysteine amino acid that is not present in naturally occurring PTX-2 protomers. The additional cysteine amino acid may participate in a disulfide bond within the antibody or antibody fragment. The additional cysteine amino acid may participate in a disulfide bond with another cysteine of the PTX-2 protomer.

In certain aspects, the antibody or antibody fragment domain of the PTX-2 protomer is a known therapeutic agent. The antibody or antibody fragment may be an anti-tissue inhibitor of matrix metalloproteinase (TIMP) antibody or antibody fragment or an anti-tumor necrosis factor (TNF) antibody or antibody fragment. The antibody or antibody fragment may be an anti-inflammatory antibody or antibody fragment.

In certain aspects, the antibody fragment domain of the PTX-2 protomer is selected from single chain variable fragments (scFv), camelid VHH proteins, or adnexins.

In certain aspects, the disclosure provides pharmaceutical preparations comprising a PTX-2 protomer as described herein and one or more pharmaceutically acceptable carriers or excipients.

In certain aspects, the disclosure provides a method for preparing a functionalized PTX-2 pentamer comprising i) introducing a first plasmid at least including a nucleotide sequence for a first functionalized PTX-2 protomer into an expression system, wherein the first plasmid at least comprises a nucleotide sequence substantially similar to a nucleotide sequence for naturally occurring PTX-2 protomer and a nucleotide sequence for one or more first antibodies or antibody fragments; ii) expressing the first functionalized PTX-2 protomer; and iii) isolating functionalized PTX-2 pentamers, purifying functionalized PTX-2 pentamers, or combinations thereof from the expression system. In some cases, the methods further comprises i) introducing a second plasmid into the expression system, wherein the second plasmid at least includes a nucleotide sequence for a second functionalized PTX-2 protomer, wherein the second plasmid at least comprises a nucleotide sequence substantially similar to a nucleotide sequence for naturally occurring PTX-2 protomer and a nucleotide sequence for one or more second antibodies or antibody fragments; and ii) inducing co-expression of the first functionalized PTX-2 protomer and the second functionalized PTX-2 protomer, the naturally occurring PTX-2 protomer, or combinations thereof. In some cases, the method further comprises i) introducing a wild type plasmid into the expression system, wherein the wild type plasmid at least comprises a nucleotide sequence substantially similar to a nucleotide sequence for naturally occurring PTX-2 protomer; and ii) inducing co-expression of the first functionalized PTX-2 protomer, the second functionalized PTX-2 protomer, the naturally occurring PTX-2 protomer, or combinations thereof. In some embodiments, the first plasmid, the second plasmid, the wild type plasmid or combinations thereof further comprise an inducible promoter positioned to control expression of the functionalized PTX-2 protomer, the naturally occurring PTX-2 protomer or any combination thereof. In some cases, the step of inducing expression comprises controlling expression of the first functionalized PTX-2 protomer, the second functionalized PTX-2 protomer, the naturally occurring PTX-2 protomer, or combinations thereof such that the first functionalized PTX-2 protomer, the second functionalized PTX-2 protomer, and/or the naturally occurring PTX-2 protomer are not expressed in equal proportions. In certain cases, controlling expression is carried out by means of controlling the copy number of the first plasmid, the second plasmid, the wild type plasmid or combinations thereof in cells of the expression system. In certain cases, controlling expression is carried out by means of an inducible promoter on the first plasmid, the second plasmid, the wild type plasmid or combinations thereof.

In certain aspects, the disclosure is directed to methods for treating a patient comprising administering an effective amount of at least one PTX-2 pentamer or protomer disclosed herein to a patient in need of treatment. In some embodiments, the PTX-2 pentamer or protomer of the disclosure is administered to reduce inflammation in a patient.

In certain aspects, the disclosure is directed to methods of preparing a colloid of PTX-2 comprising i) isolating PTX-2 pentamers, purifying PTX-2 pentamers, or combinations thereof; and ii) adding calcium to the isolated and/or purified PTX-2 pentamers. In some embodiments, the colloid is used in the preparation of a topical formulation of PTX-2.

In certain aspects, the disclosure is directed to methods of treating or preventing inflammation or an inflammatory-related disorder in a patient comprising administering an effective amount of at least one PTX-2 pentamer or protomer disclosed herein to a patient in need of thereof. In some cases the inflammatory related disorder is selected from: psoriasis, Crohn's disease, autoimmune diseases such as ankylosing spondylitis, psoriatic arthritis, rheumatoid arthritis, ulcerative colitis, lupus erythematosus, and sarcoidosis. In certain cases, the disclosure provides methods of treating or preventing inflammation or an inflammatory-related disorder in a patient comprising administration of an effective amount anti-TNF-α-PTX-2 protomer or pentamers thereof. In certain cases, the anti-TNF-α-PTX-2 protomer comprises an amino acid sequence that is at least 80%, 85%, 90%, 95%, 97%, 98%, 99%, or 100% identical to the amino acid sequence of SEQ ID NO:2.

In certain aspects, the disclosure is directed to methods of treating or preventing a PTX-2-responsive disorder in a patient comprising administering an effective amount of at least one PTX-2 pentamer or protomer disclosed herein to a patient in need of thereof. In some cases the PTX-2-responsive disorder is selected from: fibrosis or a fibrosis-related disease, a hypersensitivity disorder, an autoimmune disorder, or mucositis. In certain cases, the disclosure provides methods of treating or preventing a PTX-2-responsive disorder in a patient comprising administration of an effective amount anti-TNF-α-PTX-2 protomer or pentamers thereof. In certain cases, the anti-TNF-α-PTX-2 protomer comprises an amino acid sequence that is at least 80%, 85%, 90%, 95%, 97%, 98%, 99%, or 100% identical to the amino acid sequence of SEQ ID NO:2.

In certain aspects, the disclosure is directed to methods of treating or preventing inflammation or an inflammatory-related disorder and a PTX-2-responsive disorder in a patient comprising administration of an effective amount of at least one PTX-2 pentamer or protomer disclosed herein to a patient in need of thereof. In some cases the inflammatory related disorder is selected from psoriasis, Crohn's disease, autoimmune diseased such as ankylosing spondylitis, psoriatic arthritis, rheumatoid arthritis, ulcerative colitis, lupus erythematosus, and sarcoidosis and the PTX-2-responsive disorder is selected from: fibrosis or a fibrosis-related disease, a hypersensitivity disorder, an autoimmune disorder, or mucositis. In preferred embodiments, the disclosure provides methods of treating or preventing inflammation or an inflammatory-related disorder in a patient and a PTX-2-responsive disorder comprising administration of an effective amount anti-TNF-α-PTX-2 protomer or pentamers thereof. In certain cases, the anti-TNF-α-PTX-2 protomer comprises an amino acid sequence that is at least 80%, 85%, 90%, 95%, 97%, 98%, 99%, or 100% identical to the amino acid sequence of SEQ ID NO:2.
**Figure 1** shows the X-ray crystal structure of the pentraxin-2 (PTX-2) pentamer. The N- and C-termini are as indicated and the spheres represent bound Ca⁺².
**Figure 2** shows diagrams of PTX-2 (short pentraxin) in comparison with PTX-3 (long pentraxin).
**Figure 3A** and **3B** show various exemplary configurations of functionalized PTX-2 pentamers.
**Figure 4** is a schematic of one exemplary method for preparing functionalized PTX-2 pentamers. Up to 5 antibody/protein SAP fusions can be combined to create pentameric complexes with combinations of catalytic, binding, and/or therapeutic activities.
**Figure 5** shows a plot of the % of PTX-2 remaining in solution after addition of calcium.
**Figure 6** is an exemplary size-exclusion high pressure liquid chromatography (SE-HPLC) plot of a purified PTX-2 anti-TNFα fusion protein showing no degradation after 5 freeze/thaw cycles.
**Figure 7** is a photograph of an exemplary sodium dodecyl sulfate polyacrylamide gel electrophoresis (SDS-PAGE) gel of a purified PTX-2 anti-TNFa fusion protein showing no degradation after 5 freeze/thaw cycles.
**Figure 8** is an exemplary liquid chromatography-mass spectrometry (LCMS) plot showing the species of PTX-2 anti-TNFα fusion proteins after purification.
**Figure 9** is an exemplary plot showing inhibition of macrophage derived chemokine (MDC) production in peripheral blood mononuclear cells (PBMCs) as a result of exposure to native PTX-2 (●) or a PTX-2 anti-TNFα fusion protein (▼).
**Figure 10** is an exemplary plot showing inhibition of IL-8 production in macrophage cells as a result of exposure to native PTX-2 (●) or a PTX-2 anti-TNFα fusion protein (▼).
**Figure 11** is an exemplary plot showing the inhibition of TNFα activity in L929 cells as a result of exposure to native PTX-2 (●), a PTX-2 anti-TNFα fusion protein (▼), or an anti-TNFα antibody, Remicade (■).
**Figure 12** is an exemplary plot showing the clearing of native PTX-2 (●) or a PTX-2 anti-TNFα fusion protein (▼) from the blood of rats over a 24 hour period.

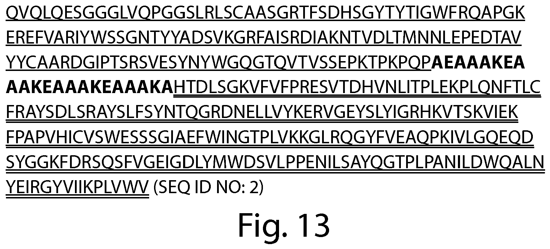

**Figure 13** shows the amino acid sequence of the anti-TNF-α-VHH3-PTX-2 protomer (SEQ ID NO:2). The anti-TNF-α-VHH3 domain is underlined, the linker region is indicated by bold font, and the PTX-2 domain is double underlined.

Before the present compositions and methods are described, it is to be understood that this disclosure is not limited to the particular processes, compositions, or methodologies described, as these may vary. It is also to be understood that the terminology used in the description is for the purpose of describing the particular versions or embodiments only, and is not intended to limit the scope of the present disclosure which will be limited only by the appended claims. Unless defined otherwise, all technical and scientific terms used herein have the same meaning as commonly understood by one of ordinary skill in the art. It must also be noted that as used herein and in the appended claims, the singular forms "a", "an", and "the" include plural reference unless the context clearly dictates otherwise. Thus, for example, reference to a "cell" is a reference to one or more cells and equivalents thereof known to those skilled in the art, and so forth.

As used herein, the term "about" means plus or minus 10% of the numerical value of the number with which it is being used. Therefore, about 50% means in the range of 45%-55%.

As used herein, the term "substantially" means being largely but not wholly what is specified. For example, the term "substantially similar" with regard to a nucleotide sequence indicates that the sequence is largely identical to another reported sequence for the same protein or peptide; however, the nucleotide sequence may include any number of variations or mutations that do not affect the structure or function of the resulting protein.

"Administering" when used in conjunction with a therapeutic means to administer a therapeutic directly into or onto a target tissue or to administer a therapeutic to a patient, whereby the therapeutic positively impacts the tissue to which it is targeted. Thus, as used herein, the term "administering", when used in conjunction with a PTX-2 scaffold can include, but is not limited to, providing a PTX-2 scaffold to a subject systemically by, for example, intravenous injection, whereby the therapeutic reaches the target tissue. "Administering" a composition may be accomplished by, for example, injection, oral administration, topical administration, or by these methods in combination with other known techniques. Such combination techniques include heating, radiation, ultrasound and the use of delivery agents.

"Providing," when used in conjunction with a therapeutic, means to administer a therapeutic directly into or onto a target tissue, or to administer a therapeutic to a patient whereby the therapeutic positively impacts the tissue to which it is targeted.

The term "animal" as used herein includes, but is not limited to, humans and non-human vertebrates such as wild, domestic and farm animals.

The term "improves" is used to convey that the present disclosure changes either the characteristics and/or the physical attributes of the tissue to which it is being provided, applied or administered. The term "improves" may also be used in conjunction with a diseased state such that when a diseased state is "improved" the symptoms or physical characteristics associated with the diseased state are diminished, reduced or eliminated.

The term "inhibiting" generally refers to prevention of the onset of the symptoms, alleviating the symptoms, or eliminating the disease, condition or disorder.

"Optional" or "optionally" means that the subsequently described event or circumstance may or may not occur, and that the description includes instances where the event occurs and instances where it does not.

Throughout the specification of the application, various terms are used such as "primary," "secondary," "first," "second," and the like. These terms are words of convenience in order to distinguish between different elements, and such terms are not intended to be limiting as to how the different elements may be utilized.

As used herein, "isolated" means altered or removed from the natural state through human intervention. For example, a rhomboid protease naturally present in a living animal is not "isolated," but a synthetic rhomboid protease, or a rhomboid protease partially or completely separated from the coexisting materials of its natural state is "isolated." An isolated rhomboid protease can exist in substantially purified form, or can exist in a non-native environment such as, for example, a cell into which the rhomboid protease has been delivered.

The terms "mimetic," "peptide mimetic," and "peptidomimetic" are used interchangeably herein, and generally refer to a peptide, partial peptide or non-peptide molecule that mimics the tertiary binding structure or activity of a selected native peptide or protein functional domain (e.g., binding motif or active site). These peptide mimetics include recombinantly or chemically modified peptides, as well as non-peptide agents such as small molecule drug mimetics, as further described below.

By "pharmaceutically acceptable," "physiologically tolerable," and grammatical variations thereof, as they refer to compositions, carriers, diluents, and reagents or other ingredients of the formulation, can be used interchangeably and represent that the materials are capable of administration without the production of undesirable physiological effects such as nausea, dizziness, rash, gastric upset or other deleterious effects to the recipient thereof.

As used herein, the term "therapeutic" means an agent utilized to treat, combat, ameliorate, prevent or improve an unwanted condition or disease of a patient. In part, embodiments of the present disclosure are directed to the treatment of inflammation, obesity-related diseases, metabolic diseases, cardiovascular diseases, cerebrovascular and neurodegenerative diseases, cancer or the aberrant proliferation of cells.

The terms "therapeutically effective" or "effective," as used herein, may be used interchangeably and refer to an amount of a therapeutic composition of embodiments of the present disclosure (e.g. one or more of the peptides or mimetics thereof). For example, a therapeutically effective amount of a composition is a predetermined amount calculated to achieve the desired effect.

A "therapeutically effective amount" or "effective amount" of a composition is a predetermined amount calculated to achieve the desired result. The activity contemplated by the present methods includes both medical therapeutic and/or prophylactic treatment, as appropriate. The specific dose of a compound administered according to this disclosure to obtain therapeutic and/or prophylactic effects will, of course, be determined by the particular circumstances surrounding the case, including, for example, the compound administered, the route of administration, and the condition being treated. However, the effective amount administered can be determined by the physician in the light of the relevant circumstances including the condition to be treated, the choice of compound to be administered, and the chosen route of administration, and therefore, the above dosage ranges are not intended to limit the scope of the disclosure in any way. A therapeutically effective amount of compound of this disclosure is typically an amount such that when it is administered in a physiologically tolerable excipient composition, it is sufficient to achieve an effective systemic concentration or local concentration in the tissue.

The terms "treat," "treated," or "treating" as used herein refers to both therapeutic treatment and prophylactic or preventative measures, wherein the object is to prevent or slow down (lessen) an undesired physiological condition, disorder or disease, or to obtain beneficial or desired clinical results. For the purposes of this disclosure, beneficial or desired clinical results include, but are not limited to, alleviation of symptoms; diminishment of the extent of the condition, disorder or disease; stabilization (*i*.*e*., not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment includes eliciting a clinically significant response without excessive levels of side effects. Treatment also includes prolonging survival as compared to expected survival if not receiving treatment.

Generally speaking, the term "tissue" refers to any aggregation of similarly specialized cells which are united in the performance of a particular function.

As used herein, the terms "antibody," "antibodies," "antibody fragment," or "antibody fragments" as well as terms associated with antibodies such as a single chain variable fragment ("scFv") shall extend to all antibodies, antibody fragments, and antibody-like binding proteins including, but not limited to, camelid VHH proteins, and adnexins.

Human pentraxin-2 (hPTX-2), also called serum amyloid P (hSAP) is constitutively expressed in the liver and circulates at approximately 20-40 µg/ml in plasma as the homopentamer of 5 non-covalently-linked protomers. Human PTX-2 functions in innate resistance to microbes and in the scavenging and phagocytosis of cellular debris and appears to play a role in regulation of wound healing and fibrosis. These functions may involve (i) binding to ligands associated with microbes and cellular debris, as specified above, and various extracellular matrix proteins in a Ca²⁺-dependent manner, (ii) binding to C1q for complement activation by promoting opsonization by C3b and iC3b, (iii) binding to Fcγ receptors to initiate direct opsonization and subsequent phagocytosis or endocytosis, and (iv) subsequent regulation of monocyte function. As such, hPTX-2 molecules localized to sites of injury and repair and may target and/or concentrate in these locations through binding these molecules.

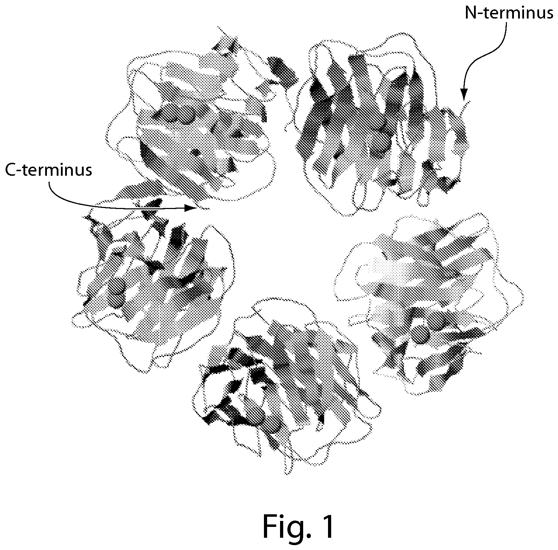

The 3D structure of hPTX-2 has been determined by X-ray crystallography (FIG. 1) and several hPTX-2 crystal structures complexed with different ligands have also been reported. The pentameric structure of hPTX-2 has 5-fold rotational symmetry and is fairly rigid with a pore. The diameter of the hPTX-2 pentamer is approximately 100Å, and the central pore is 20 Å in diameter and 35 Å deep. Each protomer is constructed of antiparallel β-strands arranged in two sheets, with a hydrophobic core with a jellyroll topology. The hPTX-2 pentamer has 2 faces, an A-face, which possesses five α helices, one on each protomer, and a B face with 5 sets of double calcium-binding sites. The B-face is thought to provide a calcium-dependent ligand binding face, and several calcium-dependent ligands that bind the B-face have been identified, including phosphorylethanolamine, DNA, heparan sulfate, dermatan sulfate and dextran sulfate, laminin and collagen IV. The A-face of hPTX-2 also appears to bind molecules such as C1q and may mediate phagocytosis through binding to Fcγ receptors. Each protomer may be glycosylated at Asn32, a single site.

N- and C-termini are solvent accessible and are located on the inner edge of each protomer molecule as indicated in **FIG. 1**. The N- terminus is located on the outer edge of each protomer and on the perimeter of the ring formed by the 5 protomers. The C-terminus is located more toward the inner perimeter and pore of the pentamer ring but is directed outward toward the A face. N- and C-termini within one protomer are about 25 Å apart. The termini do not appear to be involved in subunit interactions and they are away from the glycan chain attached at Asn32. The subunits of hPTX-2 are held together non-covalently with approximately 15% of the surface of each subunit involved in these interactions. These extensive interactions account for the considerable stability of the hPTX-2 pentamer.

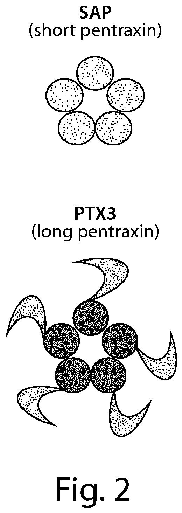

Pentraxin-2 is a member of the pentraxin family of proteins that is related to other short-pentraxins, such as pentraxin-1 (PTX-1, also called C-reactive protein, CRP), and long pentraxins, such as PTX-3. As illustrated in **FIG. 2**, all pentraxins share a basic pentraxin domain (circles), and in short pentraxins, this basic pentraxin domain defines the entire protein. In contrast, long pentraxins include the basic pentraxin domain (circles) as well as a structurally and functionally unrelated N-terminal domain that can be approximately equal in length to the pentraxin domain (flared extensions). The N-terminal domain of PTX-3 is predicted to be a α-helical coiled coil that has been shown to bind fibroblast growth factor 2. The C-terminal pentraxin domain of PTX-3 is able to bind C1q like short pentraxins PTX-2 and CRP, and although no crystal structures of the long pentraxins have been obtained, the projected structure of PTX-3 pentraxin domain bears a strong similarity to both PTX-1 and PTX-2. Long pentraxins, therefore, appear to have two separate functional domains creating the possibility that additional protein domains, peptides or other non-protein molecules with different functional properties from PTX-2 may be attached to the N- or C-terminus of PTX-2 while retaining the overall pentraxin domain and pentameric structure and function of PTX-2.

Additionally, the pentameric nature of hPTX-2 molecules results in increased avidity of interactions with other molecules. This may be advantageous over molecular interactions of simpler nature that involve the close availability of only one binding site from each molecule and may lead to increased affinity for the molecule and more effective response at lower delivered molecule concentrations. The pentameric nature of hPTX-2 also leads to possibility of cross-linking of, for example, Fcγ receptors on the cell surface, which may affect cellular responses. These properties, together with properties of hPTX-2 that localize it to sites of injury and repair, establish the potential of hPTX-2 to be used as a "scaffold" molecule to deliver other molecular entities effectively, as therapeutic, prophylactic or diagnostic agents. Other molecular entities added to hPTX-2 may also add new functional and/or delivery properties to such a fusion protein molecule.

Described is the use of pentraxin-2 (PTX-2) as a scaffold molecule for antibody and antibody fragment based therapeutic agents. In such cases, one or more antibody and antibody fragment may be linked to a PTX-2 protomer to form a "functionalized PTX-2 protomer," and these functionalized PTX-2 protomers may be combined to form functionalized PTX-2 pentamers. Thus, some cases are directed to functionalized PTX-2 protomers, and other cases are directed to functionalized PTX-2 pentamers. Yet other cases are directed to pharmaceutical compositions that include functionalized PTX-2 protomers and functionalized PTX-2 pentamers, and methods for using functionalized PTX-2 protomers and functionalized PTX-2 pentamers for the treatment of various disorders and diseases. Still other cases are directed to methods for making functionalized PTX-2 protomers and functionalized PTX-2 pentamers having the structure described herein.

An "PTX-2 fusion protein" as used herein is defined as a molecule presenting combined structural and functional properties of PTX-2 with additional molecular entities bound or attached to PTX-2 that provide further structural and functional properties. A "scaffold" function for PTX-2 may make use of the rigid pentameric structure of PTX-2, the rotational symmetry and dimensions of the pentamer, the availability of both N- and C-termini on each protomer, as well as other specific features of its structure including its Ca²⁺ binding sites and Ca²⁺-dependent ligand binding sites, C1q and FcγR binding sites, central pore, and glycosylation site.

The functionalized PTX-2 protomers may be arranged in numerous ways to carry out the scaffold function. For example, in some cases, one or more antibody, one or more antibody fragments, or combinations thereof can be attached to either the N-terminus or the C-terminus or both the N-terminus and C-terminus of an PTX-2 protomer. Because both the N- and C-termini of PTX-2 protomers are solvent accessible and located on the perimeter of the protomers where they are not involved in subunit interactions, the resulting PTX-2 fusion protein may retain its general function and structure in such embodiments while providing a delivery vehicle for the antibodies or antibody fragments.

In still other cases, one or more antibody, one or more antibody fragment, or a combination thereof may be engineered into an internal sequence of PTX-2, which is designed to be located on an exterior surface of the PTX-2 protomer based on modeling of known X-ray crystal structures of PTX-2. Such non-PTX-2 sequences may be provided in addition to the full sequence of PTX-2, or such non-PTX-2 sequences may be substituted for PTX-2 sequences. The PTX-2 may generally retain the overall pentraxin domain, pentameric structure, and general function of PTX-2 while the non-PTX-2 sequences may confer additional functional properties upon PTX-2.

In yet other cases, antibodies and antibody fragments may be attached to individual PTX-2 protomers or pentameric PTX-2 through chemical means such as, for example, chemical linkages or very tight binding with amino acids or carbohydrates on the surface of the PTX-2 protomers or pentameric PTX-2 or through chelation with the Ca²⁺ binding sites on each PTX-2 protomer. In further embodiments, one or more other metal binding sites may be substituted for the Ca²⁺ binding sites or otherwise engineered into the PTX-2 protomers elsewhere on the molecule to produce PTX-2 protomers or pentameric PTX-2 that bind to specific classes of antibodies and antibody fragments.

The PTX-2 described herein include PTX-2 from any source such as, for example, human PTX-2 or isomers or analogs from other vertebrate or mammalian sources. PTX-2 further encompasses PTX-2 molecules having modifications from the native PTX-2 amino acid sequence introduced by, for example, site-directed mutagenesis. Such modification may alter specific amino acids and/or other features of the molecule, while retaining the general pentameric pentraxin nature of the molecule. The "PTX-2" may be used to encompass both PTX-2 pentamers and PTX-2 protomers. "PTX-2 pentamer" or "pentameric PTX-2" refers to a protein complex at least including five PTX-2 protomers, and "PTX-2 protomer" refers to one individual protein unit of the PTX-2 pentamer.

The sequence of the mature human PTX-2 protomer is disclosed below, which corresponds to amino acids 20-223 of Gene bank Accession NO. NP_001630 (signal sequence not depicted).

HTDLSGKVFVFPRESVTDHVNLITPLEKPLQNFTLCFRAYSDL
SRAYSLFSYNTQGRDNELLVYKERVGEYSLYIGRHKVTSKVIE
KFPAPVHICVSWESSSGIAEFWINGTPLVKKGLRQGYFVEAQP
KIVLGQEQDSYGGKFDRSQSFVGEIGDLYMWDSVLPPENILSA
YQGTPLPANILDWQALNYEIRGYVIIKPLVWV (SEQ ID NO: 1)

In some cases, the PTX-2 protomer may be 100% identical to the native amino acid sequence of the source PTX-2 as determined using FASTDB (SEQ ID NO: 1), and in other cases, the PTX-2 protomer may have an amino acid sequence that is at least 60%, at least 70%, at least 80%, at least 85%, at least 90%, at least 95%, at least 97%, at least 98%, at least 99% identical to SEQ ID NO:1. In particular cases, non-evolutionarily conserved amino acids may be included in the non-identical amino acids. "Non-evolutionarily conserved amino acids" as used herein may refer to amino acids that are variable as in when the primary amino acid sequence of evolutionarily-related, orthologous, species, e.g., those in the same order, are compared. In various cases set forth below, PTX-2 amino acids that may be mutated or modified may generally be those amino acids that are not evolutionarily conserved. For example, an PTX-2 protomer having an amino acid sequence that is at least 95% identical to human PTX-2 (SEQ ID NO: 1) may have non-identical residues at positions where human PTX-2 and other vertebrate PTX-2 differ.

In certain cases, the non-identical amino acids may have similar chemical properties to the native amino acid to effect a "conservative substitution." In particular, amino acids that are known to have similar properties include: E, D, N, Q; H, K, R; Y, F and W; I, L, V, M, C, A; and S, T, C, P, A, and conservative substitutions are the replacements, one for another, of aliphatic amino acids Ala, Val, Leu, and Ile, basic amino acids Lys and Arg, acidic amino acids Asp and Glu, hydroxyl containing amino acids Ser and Thr, amide containing amino acids Asn and Gln, and aromatic amino acids Phe and Tyr. Additional guidance concerning which amino acid changes are likely to be phenotypically silent can be found in Bowie et al., Science 247:1306-1310 (1990). Polypeptides sharing at least 95% identity with SEQ ID NO: 1 may include polypeptides having conservative substitutions in these areas of divergence.

In some cases, structural features of PTX-2 protomers that confer specific functions such as, for example, binding to FcγR or Ca²⁺-dependent ligand binding, can be mutated to eliminate these features. In such cases, eliminating structural features that are not necessary for the intended function may eliminate potential interference with the function or targeting of the PTX-2 fusion protein. In other cases, other changes to the PTX-2 protomer such as changes to the amino acid sequence at the N- or C-terminus or an internal sequence that forms part of a surface-exposed loop may be introduced by, for example, site-directed mutagenesis and may provide additional features to the PTX-2 fusion protein such as, for example, improving solubility or adding an amino acid or modified amino acid that can be used to attach active agents. In particular cases, structural features of PTX-2 such as, for example, FcγR binding or Ca²⁺-dependent ligand binding can be eliminated by selectively mutating amino acids involved in these functions. In some cases, such modified PTX-2 protomers or pentameric PTX-2 may be used when targeting of another moiety as conferred by an attached active agent is desired. Additionally, selectively eliminating or reducing FcyR binding or Ca²⁺-dependent ligand binding may reduce the ability of PTX-2 to accumulate in areas of inflammation allowing greater retention of the PTX-2 fusion protein in circulation. Thus, modified PTX-2 protomers may be useful when greater retention in the circulatory environment is desired, or when competition with endogenous PTX-2 binding would be undesirable.

In such cases, the addition of secondary protein sequences including antibody and antibody fragment sequences used to provide a therapeutic activity for the PTX-2 and any site-directed mutagenesis or deletion to the PTX-2 protomer may be located such that the pentameric structure of PTX-2 is substantially maintained. Engineering of such fusion proteins may be guided based on available PTX-2 X-ray crystal structures, together with knowledge of key residues involved in intraprotomer and extramolecular binding interactions. Thus, sequences and individual amino acid residues known to be involved in the interaction between protomers, and generally those necessary for the formation of the flattened jellyroll pentraxin structure can be identified and secondary protein sequences may be added outside such regions so that PTX-2 protomer fusion protein can associate into pentamers. For example, site-directed mutagenesis may be carried out at amino acids within the Ca²⁺ binding, or receptor or ligand binding domains of the PTX-2 protomer to produce a PTX-2 fusion protein with the desired characteristics, and in other embodiments, the Ca²⁺ binding, or receptor or ligand binding domains may be completely or substantially deleted.

In still other cases, "PTX-2 protomer" may encompass functional fragments and fusion proteins that include any of the portions of a PTX-2 protomer. A "functional fragment" of PTX-2 may include one or more portions of the PTX-2 protomer or domains that retain the ability to carry out one or more functions associated with the PTX-2 protomer as a whole. For example, a functional fragment may include only the portions of the PTX-2 protomer necessary for pentamer assembly, and in other cases, a functional fragment may include the portions of the PTX-2 protomer necessary for pentamer assembly and a portion of the PTX-2 protomer necessary for ligand binding or Ca²⁺ binding. In certain cases, such functional fragments may be further modified to include an active agent thereby creating a functionalized PTX-2 protomer functional fragment.

Yet other cases relate to PTX-2 variants. "PTX-2 variant" is intended to refer to a protein having significantly similar primary amino acid sequence and/or structural similarity to PTX-2 protomer or from two to five assembled PTX-2 protomers that demonstrate one or more improved features as compared to the human PTX-2 pentamer including, but not limited to, increased water solubility, increased plasma half-life, increased *in vitro* stability, increased *in vivo* stability, and increased potency.

Generally, a PTX-2 protomer or functional fragment of an PTX-2 protomer useful in embodiments may be soluble in aqueous solutions at biologically-relevant temperatures, pH, and osmolarity levels. The protomers that non-covalently associate together to form PTX-2 may have identical amino acid sequences and/or post-translational modifications or, alternatively, individual protomers may have different sequences and/or modifications.

In some cases, an antibody or antibody fragment may be attached to PTX-2 protomers using recombinant technology to add or substitute sequences at the N-terminus and/or C-terminus of the PTX-2 protomer to create fusion proteins. In such cases, one or more antibody or antibody fragment may be introduced at the Nor C- terminus of the PTX-2 protomer, and in some cases, two or more antibodies and antibody fragments may be introduced consecutively such that each antibody or antibody fragment neighbors at least one other antibody or antibody fragments as in pearls on a string. In other cases, one or more antibody or antibody fragment may be encoded on each of the N- and C-termini of the PTX-2 protomer. For example, in particular embodiments, a first antibody or antibody fragment amino acid sequence may be introduced at the C-terminus of the PTX-2 protomer and a second antibody or antibody fragment amino acid sequence may be introduced at the N-terminus of the same PTX-2 protomer.

In other cases, the antibody or antibody fragment amino acid sequence added to a PTX-2 protomer may include linkers that separate added antibody or antibody fragment amino acid sequences from the necessary structural units of the PTX-2 protomer. Such a linker may additionally separate or bring into closer proximity the non-PTX-2 moieties, and/or encourage protein folding of the antibody, antibody fragment, or PTX-2 protomer. Any of the numerous linker sequences known in the art may be used in embodiments. Such linkers may be flexible linkers and may include repeating units of amino acid sequence GGGGS, (GGGGS)ₙ wherein n can be from 1 to 5. The linker may be GGGGSGGGGSGGGGS, which is commonly used to separate the light and heavy chains in scFvs. Shorter versions of such linkers having, for example, an n that is less than 3, may be used. In still other embodiments, the linker may be a rigid linker, which may be based on alpha-helix forming sequences such as, for example, (PAPAP)ₙ where n can be from 1 to 5. The length of either flexible or rigid linkers may be adjusted to provide desired proximity between, for example, the PTX-2 protomer and the antibody or antibody fragment amino acid sequences on neighboring protomers, or to ensure that the functional and/or physical properties of PTX-2 are maintained.

Cysteine pairs may be incorporated to form intrachain or interchain disulfides to stabilize loops or domains and to aid in presentation of antibody or antibody fragment on the PTX-2 protomer. For example, a cysteine may be incorporated into the PTX-2 protomer by, for example, site-directed mutagenesis, that forms a disulfide bond with a cysteine in the antibody or antibody fragment amino acid that are attached to PTX-2 protomers. Site-directed mutagenesis may be used to add a disulfide bond to stabilize portions of the PTX-2 protomer that may have formed interactions that are lost as a result of deletions or substitutions in portions of PTX-2 where FcyR binding or Ca²⁺-dependent ligand binding occur when these functions are eliminated.

PTX-2 pentamer fusion proteins may be composed of functionalized PTX-2 protomers each having the same attached antibodies or antibody fragments, and in other cases, pentameric PTX-2 fusion proteins may include functionalized PTX-2 protomers having different attached antibodies or antibody fragments. Pentameric PTX-2 fusion proteins can include a combination of functionalized and unfunctionalized PTX-2 protomers, and the functionalized PTX-2 protomers of such pentameric PTX-2 fusion proteins may have the same or different attached antibodies or antibody fragments. Functionalized PTX-2 protomers having different attached antibodies or antibody fragments may be combined into PTX-2 pentamers. As will be understood by the skilled artisan, pentameric PTX-2 pentamers having any combination of functionalized PTX-2 protomers and unfunctionalized PTX-2 protomers can be created, and availability of a specific antibody and antibody fragment can be effected by modifying the proportions of the functionalized protomer in the mixture of functionalized protomers and unfunctionalized protomers used to prepare the functionalized pentameric PTX-2. For example, a mixture of functionalized protomers and unfunctionalized protomers prepared at a ratio of 1 functionalized PTX-2 protomer to 4 unfunctionalized PTX-2 protomer will create PTX-2 pentamers having on average 1 functionalized PTX-2 protomer and 4 unfunctionalized PTX-2 protomers.

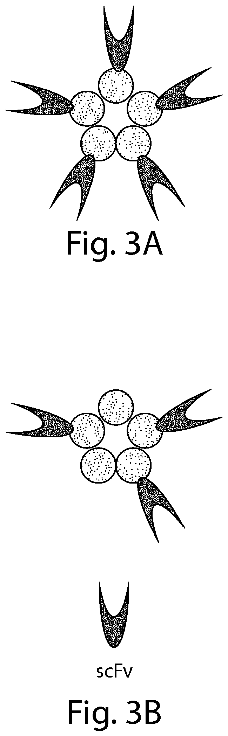

Examples of functionalized PTX-2 pentamers include PTX-2 pentamers with potential therapeutic implications that have been designed to meet current medical needs. For example, as shown in FIG. 3, each protomer of the PTX-2 pentamer may be functionalized and may include the antibody and antibody fragment introduced at the N- or C-terminus. In particular, FIG. 3A shows PTX-2 protomers that include an scFv fragment drawn approximately to scale, such as, an anti-TNF-α scFv, which may be used as an anti-inflammatory agent, and the PTX-2 pentamers may include both functionalized protomers and unfunctionalized protomers as shown in FIG. 3B. The antibody fragment shown in FIG. 3 is drawn to scale and is about the same molecular size as the N-terminal extension on PTX-2.

In certain aspects, the disclosure provides functionalized PTX-2 protomers, as well as pentamers comprising the same, that binds to TNF-α. In certain cases a functionalized PTX-2 protomer that binds to TNF-α comprises an anti-TNF-a antibody or antigen-binding fragment thereof. For example, anti-TNF-α-PTX-2 protomers of the disclosure may comprise the TNF-α antibody infliximab or an antigen-binding portion thereof. A functionalized anti-TNF-α-PTX-2 protomer, as well as pentamers comprising the same, of the disclosure may comprise an amino acid sequence that is at least 80%, 85%, 90%, 95%, 97%, 98%, 99%, or 100% identical to the amino acid sequence of SEQ ID NO:2. Optionally, a functionalized anti-TNF-α-PTX-2 protomer, as well as pentamers comprising the same, of the disclosure includes one or more modified amino acid residues selected from: a glycosylated amino acid, a PEGylated amino acid, a farnesylated amino acid, an acetylated amino acid, a biotinylated amino acid, an amino acid conjugated to a lipid moiety, and an amino acid conjugated to an organic derivatizing agent.

A functionalized PTX-2 protomer that binds to TNF-α, as well as pentamers comprising the same, may be formulated as pharmaceutical preparations comprising the functionalized PTX-2 protomer that binds to TNF-α and a pharmaceutically acceptable carrier. Such pharmaceutical preparations may also include one or more additional compounds such as a compound that is used to treat a related TNF-α disorder and/or inflammation in patient. In general, it is preferable that a functionalized anti-TNF-α-PTX-2 protomer, as well as pentamers comprising the same, be expressed in a mammalian cell line that mediates suitably natural glycosylation of the anti-TNF-α-PTX-2 protomer so as to diminish the likelihood of an unfavorable immune response in a patient. Human and CHO cell lines may be used, and it is expected that other common mammalian expression systems will be useful.

Described are also methods for preparing functionalized PTX-2 protomers. The antibody or antibody fragment DNA sequence can be introduced into the PTX-2 protomer DNA sequence to create a recombinant DNA sequence that produces a functionalized PTX-2 protomer when expressed. Standard molecular biology techniques can be used to create expression vectors or plasmids having an appropriate DNA sequence in an opening reading frame that will lead to the expression of the functionalized PTX-2 protomer when the expression vector is introduced into an appropriate expression system. As indicated above, the sequence of the antibody or antibody fragment may be fused to the N-terminus or C-terminus of the PTX-2 protomer and arranged such that the sequence will be in the proper orientation. A DNA sequence for a linker, such as those described above, may be positioned between the N-terminus or C-terminus and the antibody or antibody fragment sequence. In certain embodiments, two or more sequences for the same or different antibody or antibody fragment can be introduced consecutively at N-terminus or C-terminus DNA sequences, and sequences for linkers can be placed between each antibody or antibody fragment sequence. One or more antibody or antibody fragment sequence can be introduced at both the N-terminus and the C-terminus DNA sequences.

The expression vectors of various embodiments may include any number of additional sequences necessary for replication, selection, and the like, and any of the numerous such sequences known in the art may be used, and any type of promoter may be used to promote expression of the functionalized PTX-2 protomer. For example, a constitutive promoter may control expression of the functionalized PTX-2 protomer and in other embodiments, the expression vectors may further include inducible promoters arranged to control expression of the functionalized PTX-2 protomer.

The expression system may vary and can include bacterial cultures or various cell cultures, and in particular embodiments, recombinant PTX-2 protomers may be expressed in mammalian or insect cell culture systems so that proper starting glycan structure is added at Asn 32 of the PTX-2 protomer. Any number of plasmids for expression of functionalized PTX-2 protomers and naturally occurring, or wild type, PTX-2 protomer may be introduced into the expression system such that co-expression of these functionalized and unfunctionalized PTX-2 protomers will result in PTX-2 pentamers displaying different active agents or different numbers of active agents on each pentamer. Expression of the functionalized and unfunctionalized PTX-2 protomers may be controlled such that the proportion of each functionalized PTX-2 protomer and/or wild type PTX-2 protomer may be controlled. Controlling expression may be carried out by any means. For example, the proportion of each protomer in a resulting PTX-2 pentamer may be controlled by the copy number of plasmids from which the functionalized or unfunctionalized PTX-2 protomers are expressed, and expression may be controlled by a constitutive promoter. Expression may be controlled by an inducible promoter.

Following expression, PTX-2 protomers and/or pentameric PTX-2 may be isolated and purified using known techniques such as, for example, centrifugation, ultracentrifugation, salting-out, dialysis, filtration, column chromatography, and the like and various combinations thereof. The recombinant functionalized or unfunctionalized PTX-2 protomer may further include a tag such as, for example, a histidine tail, to aid in purification.

When more than one type of functionalized PTX-2 protomer is used to create the PTX-2 pentamer, expression vectors for one or more functionalized and/or unfunctionalized PTX-2 protomers may be introduced into an appropriate expression system such that the desired combination of protomers are simultaneously expressed, *i.e.* co-expressed. Appropriate purification technology may then be used to separate those PTX-2 pentamers having the desired combination of moieties from those not through, for example, inclusion of purification tags introduced recombinantly and/or through sequential use of chromatographic separations that make use of exclusive properties of each of the moieties. Design features such as certain amino acids in the PTX-2 protomer interaction areas may be introduced into specific protomers such that association of PTX-2-protomers bearing non-like moieties is promoted during assembly.

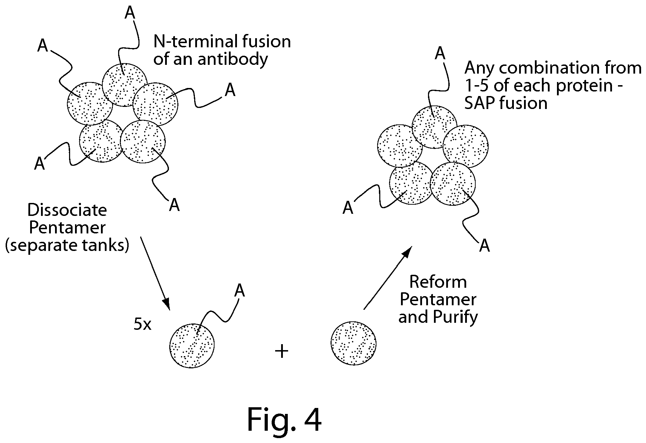

Functionalized PTX-2 pentamers may be isolated and/or purified directly following preparation as, in general, PTX-2 pentamers will form in the presence of sufficient PTX-2 protomer and/or functionalized PTX-2 protomers, and these isolated and/or purified functionalized PTX-2 pentamers may be incorporated directly into, for example, a pharmaceutical composition. Methods for preparing functionalized PTX-2 pentamers may include isolating and/or purifying functionalized PTX-2 pentamers, and then dissociating the functionalized PTX-2 pentamers into functionalized PTX-2 protomers. The separated functionalized PTX-2 protomers may then be combined with other functionalized or unfunctionalized PTX-2 protomers followed by reforming PTX-2 pentamers from the combination of PTX-2 protomers. An exemplary schematic is provided in FIG. 4. As illustrated, up to five different PTX-2 protomers can be combined resulting in an PTX-2 pentamer with up to five different attached active agents. The PTX-2 protomers combined may include from 1 to 5 functionalized PTX-2 protomers and will result in PTX-2 pentamers having less than five different active agents. Unfunctionalized PTX-2 protomers may be combined with the PTX-2 protomers having from 1 to 5 attached active agents to reduce the number of active agents associated with the PTX-2 pentamer.

In certain aspects, the disclosure provides nucleic acid encoding a functionalized anti-TNF-α-PTX-2 protomer, fragments, and function variants thereof. The subject nucleic acids may be single or double stranded. Such nucleic acids may be DNA or RNA. Described are isolated or recombinant nucleic acid sequenced that encode for an anti-TNF-α-PTX-2 protomer that is at least 80%, 85%, 90%, 95%, 97%, 98%, 99%, or 100% identical to the amino acid sequence of SEQ ID NO:2. One of skill in the art will appreciate that nucleic acid sequence that are complementary to SEQ ID NO:2 are also within the scope of this disclosure. The anti-TNF-α-PTX-2 protomer nucleic acids disclosed herein may be operably linked to a promoter for expression, and disclosure provides cells transformed with such recombinant polynucleotides. Preferably the cell is a CHO cell.

PTX-2 fusion protein may have therapeutic, prophylactic, or diagnostic use in, for example:
**Avidity:** Avidity represents the combined strength of multiple bond affinities, and enhanced avidity may improve the potency of a delivered agent by enhancing specificity through improved binding properties thereby increasing the therapeutic index for the delivered agent. Many active agents for therapeutic or prophylactic use are limited in effectiveness due to relatively low avidity for the molecules to which they bind resulting in poor effectiveness, non-specific effects, and low and possibly inadequate therapeutic index. Without wishing to be bound by theory, increased avidity for a delivered agent may be achieved by presenting the molecule on a scaffold that is multimeric such as pentameric PTX-2, thereby increasing the local concentration of the agent through increased strength of multiple bond affinities. This advantage can be used to drive a local effect that avoids a broader systemic effect, and this can result in higher specificity and possibly less toxic systemic effects. Thus, in some embodiments, PTX-2 fusion proteins may be used to increase the avidity of a therapeutic or prophylactic antibody or antibody fragment.

For example, a reduction in receptor signaling may be desirable, for example, when there is overproduction of the cognate ligand associated with a disease state. A functionalized PTX-2 may be used to block the action of the cognate ligand to treat the disease. For example, rheumatoid arthritis can be caused by overproduction of an inflammatory cytokine such as TNFα and can lead to tissue damage. A functionalized PTX-2 may directly bind to the cognate ligand to inhibit its binding to the receptor by providing an antibody or antibody fragment on the functionalized PTX-2. Functionalized PTX-2 may directly bind the ligand reducing the circulating concentration of the ligand and reducing inflammation.

**Targeting:** PTX-2 fusion proteins can also be engineered to specifically target certain other molecules or specific cells or sites *in vivo* for use in therapeutic and prophylactic applications. For example:
*PTX-2-directed targeting:* PTX-2 binds a variety of ligands that are concentrated at sites of infection, wounding, repair, and fibrosis as well as C1q component of the complement pathway and FcγR, which are expressed on the surface of a variety of immune cells, including monocytes. PTX-2 may provide a means to direct antibodies or antibody fragments to sites of infection, wounding, repair, and fibrosis, and the surface of various immune cells, and in certain embodiments, PTX-2 may allow antibodies or antibody fragments to enter these cells through FcγR internalization by endocytosis or phagocytosis. PTX-2 also binds to apoptotic cell debris and may indirectly decrease the activation of myofibroblasts responsible for excess extracellular matix formation, a hallmark of fibrosis.

*Targeting through other moieties:* One or more antibodies or antibody fragments may be included as part of the PTX-2 scaffold and may be utilized to target the resulting PTX-2 fusion protein to a particular cell or tissue type. For example, an antibody or antibody fragment may bind to a specific receptor, cell surface molecule, or to another site, such as a site of complement fixation or extracellular matrix, and direct the PTX-2 fusion protein to that specific cell-type or tissue. A PTX-2 fusion protein having attached antibodies or antibody fragments targeted to an APC surface receptor that can induce endocytosis. Upon contact with an APC, the pentameric PTX-2 fusion protein can allow internalization. A pentameric PTX-2 fusion protein may include antibodies or antibody fragments capable of binding to CD40 or another cell-surface marker involved in triggering DC maturation. Binding to a CD40 or another cell-surface marker involved in triggering DC maturation may lead to DC maturation which may result in enhanced effective anti-tumor immunity.

*Targeting to bring different cell types into close proximity:* PTX-2 fusion proteins may include antibodies or antibody fragments that may act to bring cells of the same or different types together, which may affect cell or pathway activation or inactivation and, in some embodiments, killing the contacted cells. Multiple antibodies or antibody fragments may be provided on the PTX-2 fusion protein, and each individual cell-targeting moiety may affect intracellular signaling. For example, immune cells such as T cells, B cells, NK cells, dendritic cells, neutrophils, monocytes, macrophages and the like are known to interact with each other, both through physical contacts via cell surface ligands and cell surface receptors, and soluble ligand interactions with cell surface receptors. By presenting a combination of antibodies or antibody fragments capable of binding these cell types using a PTX-2 fusion protein, interactions between the cells listed above and potentially those involving other cell types may be enhanced by improving proximity of the cells to one another.

**Neutralization of activity:** Functionalized PTX-2 fusion proteins may be used to bind to unwanted molecules circulating *in vivo* and neutralize their activity. Undesired molecules can include, but are not limited to, molecules produced by exogenous agents such as, for example, bacterial toxins or viruses, or endogenous molecules present at undesirable levels through disease processes such as, for example, inflammatory cytokines. Without wishing to be bound by theory, the multivalent antibodies or antibody fragments on a PTX-2 scaffold protein may improve avidity, which may lead to a tighter binding and more rapid and effective sequestration and neutralization of the unwanted molecules.

*Neutralization of endogenous molecules:* Raised levels of certain biological molecules such as, for example, hormones, cytokines, growth factors, and the like may contribute to disease. For example, HB-EGF levels may be raised in various cancers and TNF levels may be raised in immune related diseases. Neutralization or lowering the level of such biological molecules can be of therapeutic value, and several therapeutic agents are currently available that act to sequester circulating cytokines to reduce inflammation such as etanercept (Enbrel®), soluble recombinant TNF receptor fused to the Fc portion of an antibody, and infliximab (Remicade®), an anti-TNF antibody, which reduce the level of the inflammatory cytokine TNF in rheumatoid arthritis and other diseases.

Pentameric PTX-2 fusion proteins created from the PTX-2 scaffold may have advantages for neutralization of endogenous molecules over such therapeutic agents. For example, PTX-2 pentamers prepared from functionalized PTX-2 protomers presenting, for example, scFvs or other antibody derivatives capable of binding TNF receptor may have improved avidity and increased effectiveness over a bivalent Fc-fusion protein because the TNF receptor is a trimer and each receptor binding site could be bound by a single pentameric PTX-2 fusion protein, but would require two molecules of Enbrel or Remicade. PTX-2 protomers may include attached scFvs or other antibody-derived moieties, to produce anti-TNFα-PTX-2 fusion proteins, and these fusion proteins may also be used to treat inflammation. Without wishing to be bound by theory, both of these types of fusion proteins may more effectively neutralize trimeric soluble TNFα than bivalent Enbrel® and be more effective in anti-inflammatory 'reverse' signaling through membrane-bound trimeric TNFα than Remicade®.

Accordingly, functionalized PTX-2 protomers, as well as pentamers thereof, of the disclosure (e.g, anti-TNF-α-PTX-2 protomers) may be used to treat or prevent inflammation and/or inflammatory-related disorders in a patient. In certain aspects, the disclosure provides methods for treating TNFα-related disorders in a patient including, for example, psoriasis, Crohn's disease, autoimmune diseased such as ankylosing spondylitis, psoriatic arthritis, rheumatoid arthritis, ulcerative colitis, lupus erythematosus, and saccoidosis. In certain aspects, functionalized PTX-2 protomers of the disclosure (e.g, anti-TNF-α-PTX-2 protomers) may be used to inhibit or reduce production of IL-8 *in vitro* and *in vivo* (i.e., in a patient in need thereof).

*Neutralization of exogenous agents of infection:* In the prophylaxis and treatment of viral disease, human neutralizing antibody-based molecules may be useful where vaccines have been difficult to develop or may pose inherent safety concerns such as in infants or immuno-compromised individuals. Recently, selection of antibody fragment sequences from phage display libraries has led to the development of antibody based neutralizing agents that have been shown to be effective *in vitro* and *in vivo.* For example, human recombinant antibody fragments have been shown to effectively neutralize metapneumovirus and RSV *in vivo* and a type-common human recombinant antibody to herpes simplex virus has been shown to provide protection against herpes simplex *in vivo.* A PTX-2 pentameric fusion protein presenting PTX-2 protomers having attached recombinant antibody fragments such as, for example, scFvs or Fab moieties and other small proteins having antibody-like binding activities including, for example, camelid VHH proteins and adnexins, may have higher avidity in binding based on their higher valency resulting in a slower off rate. Thus, such PTX-2 fusion proteins may remain bound to the viral protein so long as at least one of the binding sites remains bound and may be more effective at neutralizing various viruses.

Pentameric PTX-2 fusion proteins may also induce cross-linking of viral particles into aggregates thereby reducing the number of infectious units. Therefore, pentameric PTX-2 fusion proteins presenting multiple antibody-derived fragments can both aggregate virus particles through cross-linking and neutralize the particles through high avidity binding to viral proteins. In addition, the distance between protomer antibody sequences may provide greater flexibility in meeting distance requirements for binding to at least two viral proteins because such antibody sequences can be anywhere from approximately the distance between those on neighboring protomers to across the span of the whole PTX-2 molecule or greater.

### PTX-2 responsive disorders

PTX-2 protomers, as well as pentamers thereof, of the disclosure including functionalized PTX-2 protomers may retain one or more native PTX-2 activities including, for example, the ability to inhibit monocyte differentiation into fibrocytes. Accordingly, the disclosure provides methods for treating a PTX-2-responsive disorder in a patient by administering a therapeutically effective amount of PTX-2 protomer, including functionalized PTX-2 protomers of the disclosure, to a patient in need thereof.

The PTX-2-responsive disorder may be fibrosis. The use of PTX-2 as a therapeutic treatment for fibrosis is described in U.S. Patent Application No. 2007/0243163. Fibrosis and fibrosis-related disorders that may be amenable to treatment with the subject method include, but are not limited to, collagen disease, interstitial lung disease, human fibrotic lung disease (e.g., obliterative bronchiolitis, idiopathic pulmonary fibrosis, pulmonary fibrosis from a known etiology, tumor stroma in lung disease, systemic sclerosis affecting the lungs, Hermansky-Pudlak syndrome, coal worker's pneumoconiosis, asbestosis, silicosis, chronic pulmonary hypertension, AIDS-associated pulmonary hypertension, sarcoidosis, moderate to severe asthma and the like), fibrotic vascular disease, arterial sclerosis, atherosclerosis, varicose veins, coronary infarcts, cerebral infarcts, myocardial fibrosis, musculoskeletal fibrosis, post-surgical adhesions, human kidney disease (e.g., nephritic syndrome, Alport syndrome, HIV-associated nephropathy, polycystic kidney disease, Fabry's disease, diabetic nephropathy, chronic glomerulonephritis, nephritis associated with systemic lupus, and the like), progressive systemic sclerosis (PSS), primary scalloping cholangitis (PSC), liver fibrosis, liver cirrhosis, renal fibrosis, pulmonary fibrosis, cystic fibrosis, chronic graft versus host disease, scleroderma (local and systemic), Grave's ophthalmopathy, diabetic retinopathy, glaucoma, Peyronie's disease, penis fibrosis, urethrostenosis after cystoscope, inner accretion after surgery, scarring, myelofibrosis, idiopathic retroperitoneal fibrosis, peritoneal fibrosis from a known etiology, drug-induced ergotism, fibrosis incident to benign or malignant cancer, fibrosis incident to microbial infection (e.g., viral, bacterial, parasitic, fungal, etc.), Alzheimer's disease, fibrosis incident to inflammatory bowel disease (including stricture formation in Crohn's disease and microscopic colitis), stromal cell tumors, mucositis, fibrosis induced by chemical or environmental insult (e.g., cancer chemotherapy, pesticides, or radiation (e.g., cancer radiotherapy)). In some embodiments, the fibrosis related disorder is selected from systemic or local scleroderma, keloids, hypertrophic scars, atherosclerosis, restenosis, pulmonary inflammation and fibrosis, idiopathic pulmonary fibrosis, liver cirrhosis, fibrosis as a result of chronic hepatitis B or C infection, kidney disease, heart disease resulting from scar tissue, macular degeneration, and retinal and vitreal retinopathy. In some embodiments, the fibrosis related disorder results from chemotherapeutic drugs, radiation-induced fibrosis, and injuries and burns. The fibrosis-related disorder or condition may result from post-surgical scarring, e.g., following trabeculectomy or other filtration surgery of the eye.

The PTX-2-responsive disorder may be a hypersensitivity disorder such as those mediated by Th1 or Th2 responses. Hypersensitivity related disorders that may be amenable to treatment with PTX-2 include, but are not limited to, allergic rhinitis, allergic sinusitis, allergic conjunctivitis, allergic bronchoconstriction, allergic dyspnea, allergic increase in mucus production in the lungs, atopic eczema, dermatitis, urticaria, anaphylaxis, pneumonitis, and allergic-asthma. In some embodiments, a PTX-2 protomer of the disclosure may be used to treat allergen-specific immune responses, such as anaphylaxis, to various antigens.

The PTX-2-responsive disorder may be an autoimmune disorder such as those mediated by Th1 or Th2 responses. Autoimmune related disorders that may be amenable to treatment with PTX-2 protomers of the disclosure include, but are not limited to, type I diabetes, multiple sclerosis, rheumatoid arthritis, psoriatic arthritis, autoimmune myocarditis, pemphigus, myasthenia gravis, Hashimoto's thyroiditis, Graves' disease, Addison's disease, autoimmune hepatitis, chronic Lyme arthritis, familial dilated cardiomyopathy, juvenile dermatomyositis, polychondritis, Sjogren's syndrome, psoriasis, juvenile idiopathic arthritis, inflammatory bowel disease, systemic lupus erythematosus, chronic obstructive pulmonary disease, and graft-versus-host disease.

In some cases, the PTX-2-responsive disorder is a mucositis.

In certain aspects, PTX-2 fusion proteins including functionalized PTX-2 of the disclosure can be used to treat multiple or unrelated disorders in a patient. PTX-2 fusion proteins, and pentamers thereof, including functionalized PTX-2 of the disclosure can be used to treat or prevent a PTX-2-responsive disorder (e.g., fibrosis or a fibrosis related disorder) and inflammation and/or an inflammatory-related disorder in patient (e.g., psoriasis, Crohn's disease, autoimmune diseased such as ankylosing spondylitis, psoriatic arthritis, rheumatoid arthritis, ulcerative colitis, lupus erythematosus, saccoidosis, etc.).

**Increased half-life of potential prophylactics and therapeutics:** PTX-2 fusion proteins including functionalized PTX-2 protomers having attached antibodies or antibody fragments may be useful to increase half-life of the active agent. Human PTX-2 is fairly stable in serum, and is resistant to proteolysis, especially in the presence of Ca²⁺. Therefore, attaching less stable molecules to a PTX-2 scaffold may improve the half-life of the less stable molecule. For example, the effective half-life of antibodies or antibody fragments may be increased by up to 50% or more by attaching the antibodies or antibody fragments to PTX-2 protomers.

The stability of the PTX-2 scaffolds may be increased by providing functionalized PTX-2 protomers having attached antibodies or antibody fragments that act to enhance PTX-2 stability thereby further increasing the half-life of the PTX-2 scaffold in circulation. For example, the FcRn, a receptor that is expressed on a variety of immune cells, has been shown to increase the half-life of IgGs by binding to circulating IgG and inducing endocytosis. The IgGs in the endosome are thus protected from degradation. When the immune cells are activated, endocytosed IgGs can be transported out of the cell via exocytosis and released back into the circulation. Several specific amino acid residues in the CH2 and CH3 domains of IgGs are essential for binding to the FcRn, and amino acids in the hinge region between the CH2 and CH3 domains of IgGs are different from those involved in FcγR binding. The key residues and structural features of IgG binding to FcγRs are also known. A chimeric PTX-2 scaffold that retains key components of CH2 and CH3 antibody domains, which bind FcRn while eliminating amino acids essential for FcγR binding may be prepared and may improve dosing regimen and effectiveness of PTX-2 scaffolds, while obviating any potential of the IgG Fc region (CH2-CH3) to interfere with PTX-2 binding to FcγRs.

**Diagnostics:** "Diagnostics agents" as used herein refer to molecules used in *in vitro* assays to detect the presence and concentration of endogenous molecules or exogenous molecules that are indicative of disease. Such diagnostic agents can also be used to detect the effectiveness of therapeutic or prophylactic agents, for example, by assaying for the presence and concentration of certain biomarkers indicative of recovery or antibodies in the case of a classical vaccine. Currently, many diagnostic agents include antibody reagents or detection of antibodies. The multivalency of PTX-2 fusion proteins prepared from functionalized PTX-2 protomers with attached antibodies or antibody fragments may result in improved interaction with a binding partner compared with a monovalent antigen/bivalent antibody interaction. In some cases, increased sensitivity and specificity can be provided by functionalized PTX-2 diagnostic agents that can enable development of diagnostic assays for molecules that are present in a sample at concentration levels that are too low to be detected by other means. Accordingly, described are PTX-2 fusion proteins and functionalized PTX-2 protomers that present antibody-derived proteins or antigen-based proteins that can be used as diagnostic agents in *in vitro* diagnostic assays. For example, PTX-2 fusion proteins presenting scFvs may be prepared to detect and quantify specific proteins or biomarkers or viral coat protein sequences to detect the formation of antibodies against virus.

PTX-2 scaffold-based diagnostic agents such as those described above may further include a marker or probe enabling such diagnostic agents to be used for imaging tissues or cells *in vivo.* Without wishing to be bound by theory, providing a multimeric PTX-2 scaffold-based diagnostic agent may provide greater sensitivity and higher resolution than similar antibody-based diagnostic agents. PTX-2 scaffold-based diagnostic agents may be used for *in vivo* imaging where targeting the site of disease is particularly important. PTX-2 scaffold-based diagnostic agents may be utilized to target sites where PTX-2 has a natural affinity such as areas where wounding, repair, fibrosis, and the like are occurring. There may be no need to include a targeting moiety.

PTX-2 scaffolds used as diagnostic agents may include any marker or probe known in the art including, but not limited to, fluorescent probes, nanoparticles, radionucleotides, and the like. The multivalency of a PTX-2 scaffold protein is expected to allow incorporation of multiple targeting moieties as well as detection moieties such as near-infrared fluorophores. The PTX-2 scaffold may include a near-infrared-based fluorescent probe. Near-infrared fluorescent probes offer advantages of long wavelength emission which reduces photon attenuation by living tissues, allowing for greater depth of detection and increased sensitivity, as well as improved safety and cost-effectiveness. Another advantage of fluorescent imaging is that proteins or peptides to which the fluorescent probes are attached can be designed and targeted to provide a molecular image rather than solely an anatomical or morphological image, as with some other imaging methods. For example, trastuzumab (Herceptin®) labeled with near-IR dye molecules have been used to detect levels of EGFR2 overexpression in breast cancer. PTX-2 scaffolds may include one or more photoactivatable dyes or fluorescent switch dyes that are sensitive to specific biochemical events, such as an enzyme activity. Similar uses to those described above in the examples may be PTX-2 scaffold proteins of this kind, in addition to imaging to directly detect distribution or specific location of a protein.

Certain structural features of PTX-2 such as, for example, FcγR binding or Ca²⁺-dependent ligand binding may be eliminated from PTX-2 protomers used in the preparation of diagnostic agents to eliminate binding to elements outside the intended target and/or to reduce non-specific interactions that could taint the results of diagnostic assays. Mutagenesis may be used to alter amino acids necessary for the interactions associated with the structural features or portions of the PTX-2 that perform such functions may be removed. PTX-2 scaffolds may be used to deliver a marker or probe to tissues or cells to which PTX-2 has a natural affinity such as, for example, sites of fibrosis and/or injury. PTX-2 scaffolds may be used to target other known binding partners such as, phosphatidylethanolamine, which is exposed on the surface of apoptotic cells and cellular debris. PTX-2 scaffold diagnostic agents may be useful for monitoring apoptosis in other diseases or treatments, such as in cancer or atherosclerosis.

PTX-2 scaffold proteins may be used with other imaging technologies. For example, PTX-2 scaffolds may be used to develop diagnostic agents for MRI by combining the PTX-2 scaffold with paramagnetic imaging agents. Such paramagnetic imaging agents are known in the art. Similar PTX-2 scaffolds that target cells involved in disease or diseased tissues may be prepared using such techniques and may provide useful diagnostic imaging agents that can provide morphological and biochemical information, and PTX-2 scaffolds presenting specific targeting moieties and MRI imaging agents can be used to target and monitor receptors or other biochemical markers of disease or recovery. The inherent targeting of PTX-2 to certain sites of injury and repair or certain cell types can also be used to target such a diagnostic imaging agent.

Described are pharmaceutical compositions at least including one or more functionalized PTX-2 pentamers, such as those described above, and one or more pharmaceutically acceptable carriers or excipients. Thus, in some cases, the pharmaceutical composition may be a therapeutic or prophylactic composition that includes PTX-2 scaffolds having attached antibodies or antibody fragments that are useful in the treatment or prevention of disease. In other cases, the PTX-2 scaffolds of the pharmaceutical composition may have antibodies or antibody fragments, probes or markers and may be used as a diagnostic pharmaceutical agent. Pharmaceutical formulations and pharmaceutical compositions are well known in the art, and can be found, for example, in Remington's Pharmaceutical Sciences, Mack Publishing Co., Easton, Pa., USA, which is hereby incorporated by reference in its entirety.

Pharmaceutical excipients are well known in the art and include, but are not limited to, saccharides such as, for example, lactose or sucrose, mannitol, trehalose, or sorbitol, cellulose preparations, calcium phosphates such as tricalcium phosphate or calcium hydrogen phosphate, as well as binders, such as, starch paste such as, for example, maize starch, wheat starch, rice starch, potato starch, gelatin, tragacanth, methyl cellulose, hydroxypropylmethylcellulose, sodium carboxymethylcellulose, polyvinyl pyrrolidone or combinations thereof.

Pharmaceutical formulations may include any of the functionalized PTX-2 pentamers described and embodied above, a pharmaceutically acceptable carrier or excipient, and any number of additional or auxiliary components known in the pharmaceutical arts such as, for example, binders, fillers, disintegrating agents, sweeteners, wetting agents, colorants, sustained release agents, and the like, and the pharmaceutical composition may include one or more secondary active agents. Disintegrating agents, such as starches as described above, carboxymethyl-starch, cross-linked polyvinyl pyrrolidone, agar, alginic acid or a salt thereof, such as sodium alginate and combinations thereof. Auxiliary agents may include, for example, flow-regulating agents and lubricants, such as silica, talc, stearic acid or salts thereof, such as magnesium stearate or calcium stearate, polyethylene glycol and combinations thereof. In certain embodiments, dragee cores may be prepared with suitable coatings that are resistant to gastric juices, such as concentrated saccharide solutions, which may contain, for example, gum arabic, talc, polyvinyl pyrrolidone, polyethylene glycol, titanium dioxide, lacquer solutions and suitable organic solvents or solvent mixtures and combinations thereof. In order to produce coatings resistant to gastric juices, solutions of suitable cellulose preparations, such as acetylcellulose phthalate or hydroxypropylmethyl-cellulose phthalate may also be used. In still other embodiments, dye stuffs or pigments may be added to the tablets or dragee coatings, for example, for identification or in order to characterize combinations of active compound doses. The carrier or excipient may not be dimethyl sulfoxide (DMSO). In certain embodiments, a carrier or excipient used in formulations may be ethanol at a concentration of greater than but not equal to 2.5%, such as greater than 5%, greater than 7.5%, or greater than 10%, and a carrier or excipient used in formulations may be ethanol at a concentration of less than but not equal to 2.5%.

The functionalized PTX-2 pentamers embodied above may be formulated for topical administration such as, for example, as a lotion. In such embodiments, the topical formulation may be an oil in water emulsion that may be prepared with a water or alcohol base, and in some embodiments, the water or alcohol concentration in the topical formulation may be sufficiently high to facilitate drying of the components of the formulation after application to the skin of the subject. Such topical formulations may include any components known in the art to be useful for the preparation of a topical formulation including, but not limited to, solubilizers, surfactants, coserfactants, and combinations thereof. Such topical formulations may include solubilizers such as, for example, Capryol™ 90, Capryol™ Pgmc, Labrafil® M 1944 CS, Labrafil® M 2125 CS, Labrasol®, Labrafac™, Lipophile W1 1349, Labrafac™ PG, Lauroglycol™ 90, Lauroglycol™ FCC, Plurol® Oleique CC 497, Transcutol® P, and the like and combinations thereof, surfactants such as, for example, Labrasol®, Plurol® Diisostearique, and the like and combinations thereof, cosurfactants, such as, for example, Capryol™ 90, Lauroglycol™ 90, and the like and combinations thereof. Any of the solubilizers, surfactants, and cosurfactants listed may be used in separate topical formulations or may be combined in a single topical formulation.

A topical or depot formulation may be prepared by providing calcium to a preparation of functionalized PTX-2 protomers in a solution. In such embodiments, the functionalized PTX-2 may form a colloidal suspension which can be used to produce a topical formulation. The amount of calcium added to the functionalized PTX-2 preparation may vary among embodiments and may be any amount of calcium necessary to cause the colloid to form. However, the amount of calcium may be about equal molar to the Ca²⁺ binding sites in the PTX-2 preparation or more. The effect of calcium on the PTX-2 preparation may be reversed by removing the calcium from the colloidal suspension by, for example, providing a chelating agent that is capable or removing the calcium from the suspension. Without wishing to be bound by theory, this colloid effect may be useful in preparing topical formulations and time release topical formulations as well as colloidal suspensions and time released colloidal suspensions for, for example, depot injection.

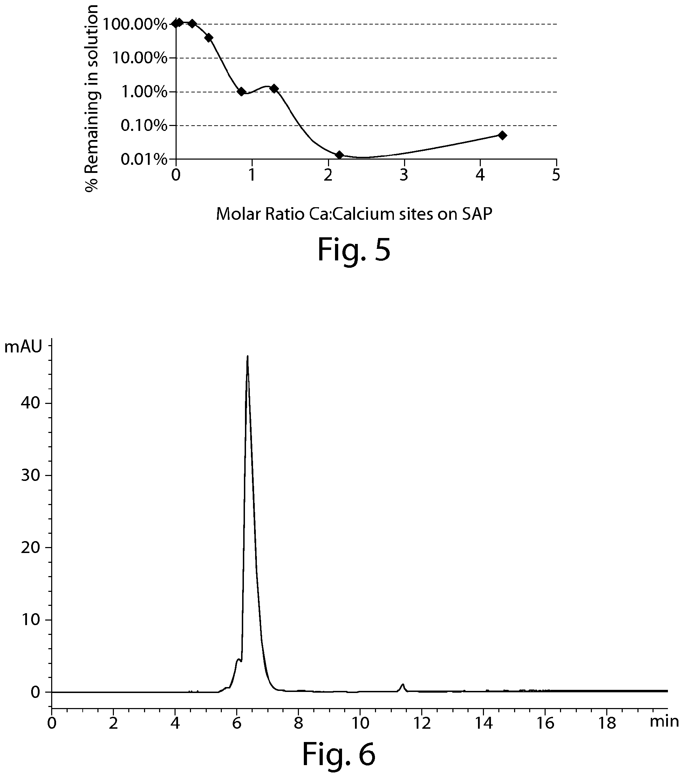

Because there are a total of 10 calcium binding sites/pentamer of PTX-2 to form a colloidal suspension from all available PTX-2 in solution, the amount of calcium must be about equimolar to the number of calcium binding sites in the PTX-2 solution. After addition of calcium, the PTX-2-Ca suspension was recovered in a pellet by centrifugation. As shown in FIG. 5, <1% of PTX-2 remains in solution when the calcium concentration is roughly equal to or greater than the concentration of calcium binding sites on PTX-2 after centrifugation. The calcium-dependent suspension is also readily reversed by addition of a molar excess of chelators such as, EDTA or citrate, and the resulting soluble PTX-2 solution recovered from the colloidal suspension does not appear to contain any increase in aggregate content relative to the starting PTX-2 solution based on SEC data.

Described are also methods for preparing a topical formulation and the topical formulations prepared by such methods. For example, in some embodiments, a topical formulation may be prepared by combining any of the compounds described above with a solubilizer and providing the solubilizer at the highest concentration possible to provide a solution. The method may further include identifying solubilizers having the best solubilizing properties, such as, highest MSA, and using these solubilizers in further steps. Such methods may further include incorporating a surfactant, or emulsifier, into the solution, and in some embodiments, the surfactant may have a low hydrophile-lipophile balance (HLB) number. The solubilized solution may be added to the surfactant very slowly, and in certain embodiments, the final concentration of solubilizer may be from about 60% to about 80% of the final solution. An alcohol may be incorporated into the topical formulation and may provide improved drying times and may aid in preserving the compound or composition. The method may include the addition of a costabilizer to produce a micro emulsion.

Pharmaceutical compositions of the disclosure can be administered to any animal, and in particular, any mammal, which may experience a beneficial effect as a result of being administered a compound of the disclosure including, but not limited to, humans, canines, felines, livestock, horses, cattle, sheep, and the like. The dosage or amount of at least one compound according to the disclosure provided pharmaceutical compositions of embodiments may vary and may depend, for example, on the use of the pharmaceutical composition, the mode of administration or delivery of the pharmaceutical composition, the disease indication being treated, the age, health, weight, etc. of the recipient, concurrent treatment, if any, frequency of treatment, and the nature of the effect desired and so on. Described are pharmaceutical compositions that include one or more compounds of the disclosure in an amount sufficient to treat or prevent diseases such as, for example, cancer. An effective amount of the one or more compounds may vary and may be, for example, from about 0.001 mg to about 1000 mg or, in other embodiments, from about 0.01 mg to about 100 mg.

The pharmaceutical compositions of the disclosure can be administered by any means that achieve their intended purpose. For example, routes of administration encompassed by the disclosure include, but are not limited to, subcutaneous, intravenous, intramuscular, intraperitoneal, buccal, subconjunctival, intravitreal or other routes, rectally, parenterally, intrasystemically, intravaginally, topically (as by powders, ointments, drops or transdermal patch), inhaled (nebulized or dry-powder), oral or nasal spray are contemplated in combination with the above described compositions.

Formulations for parenteral administration may include one or more compounds of the disclosure in water-soluble form, for example, water-soluble salts, alkaline solutions, and cyclodextrin inclusion complexes in a physiologically acceptable diluents which may be administered by injection. Physiologically acceptable diluents of such embodiments, may include, for example, sterile liquids such as water, saline, aqueous dextrose, other pharmaceutically acceptable sugar solutions; alcohols such as ethanol, isopropanol or hexadecyl alcohol; glycols such as propylene glycol or polyethylene glycol; glycerol ketals such as 2,2-dimethyl-1,3-dioxolane-4-methanol; ethers such as poly(ethyleneglycol)400; pharmaceutically acceptable oils such as fatty acid, fatty acid ester or glyceride, or an acetylated fatty acid glyceride. Formulations suitable for parenteral administration may additionally include one or more pharmaceutically acceptable surfactants, such as a soap or detergent; suspending agent such as pectin, carbomers, methylcellulose, hydroxypropylmethylcellulose, or carboxymethylcellulose; an emulsifying agent; pharmaceutically acceptable adjuvants or combinations thereof. Additional pharmaceutically acceptable oils which may be useful in such formulations include those of petroleum, animal, vegetable or synthetic origin including, but not limited to, peanut oil, soybean oil, sesame oil, cottonseed oil, olive oil, sunflower oil, petrolatum, and mineral oil; fatty acids such as oleic acid, stearic acid, and isostearic acid; and fatty acid esters such as ethyl oleate and isopropyl myristate. Additional suitable detergents include, for example, fatty acid alkali metal, ammonium, and triethanolamine salts; cationic detergents such as dimethyl dialkyl ammonium halides, alkyl pyridinium halides, and alkylamine acetates; and anionic detergents, such as alkyl, aryl, and olefin sulfonates, alkyl, olefin, ether and monoglyceride sulfates, and sulfosuccinates. Non-ionic detergents including, but not limited to, fatty amine oxides, fatty acid alkanolamides and polyoxyethylenepolypropylene copolymers or amphoteric detergents such as alkyl-β-aminopropionates and 2-alkylimidazoline quaternary salts, and mixtures thereof may be useful in parenteral formulations of the disclosure.

Buffers, preservatives, surfactants and so on may also be added to formulations suitable for parenteral administration. For example, suitable surfactants may include polyethylene sorbitan fatty acid esters, such as sorbitan monooleate, and the high molecular weight adducts of ethylene oxide with a hydrophobic base, formed by the condensation of propylene oxide with propylene glycol.

Pharmaceutical compositions for parenteral administration may contain from about 0.5 to about 25% by weight of one or more of the functionalized PTX-2 pentamers and from about 0.05% to about 5% suspending agent in an isotonic medium. The injectable solution should be sterile and should be fluid to the extent that it can be easily loaded into a syringe. In addition, injectable pharmaceutical compositions may be stable under the conditions of manufacture and storage and may be preserved against the contaminating action of microorganisms such as bacteria and fungi.

Topical administration includes administration to the skin or mucosa, including surfaces of the lung and eye. Compositions for topical administration, may be prepared as a dry powder which may be pressurized or non-pressurized. In non-pressurized powder compositions, the active ingredients in admixture are prepared as a finely divided powder. At least 95% by weight of the particles of the admixture may have an effective particle size in the range of 0.01 to 10 micrometers. The finely divided admixture powder may be additionally mixed with an inert carrier such as a sugar having a larger particle size, for example, of up to 100 micrometers in diameter. Alternatively, the composition may be pressurized using a compressed gas, such as nitrogen or a liquefied gas propellant. When a liquefied propellant medium is used, the propellant may be chosen such that the compound and/or an admixture including the compound do not dissolve in the propellant to any substantial extent. A pressurized form of the composition may also contain a surface-active agent. The surface-active agent may be a liquid or solid non-ionic surface-active agent or may be a solid anionic surface-active agent, which may be in the form of a sodium salt.

Compositions for rectal or vaginal administration may be prepared by mixing the compounds or compositions of the disclosure with suitable non-irritating excipients or carriers such as for example, cocoa butter, polyethylene glycol or a suppository wax. Such carriers may be solid at room temperature but liquid at body temperature and therefore melt in the rectum or vaginal cavity and release the drugs.

The compounds or compositions of the disclosure can be administered in the form of liposomes. Liposomes are generally derived from phospholipids or other lipid substances that form mono- or multi-lamellar hydrated liquid crystals when dispersed in an aqueous medium. Any non-toxic, physiologically acceptable and metabolizable lipid capable of forming liposomes can be used, and in particular embodiments, the lipids utilized may be natural and/or synthetic phospholipids and phosphatidyl cholines (lecithins). Methods to form liposomes are known in the art (see, for example, Prescott, Ed., Meth. Cell Biol. 14:33 (1976), which is hereby incorporated by reference in its entirety). Compositions including one or more compounds of the disclosure in liposome form can contain, for example, stabilizers, preservatives, excipients and the like.

One or more compounds of the disclosure may be formulated for *in vitro* use. The composition of the disclosure may include one or more compounds presented herein above in a carrier that is suitable for an assay. Such carriers may be in solid, liquid or gel form and may or may not be sterile. Examples of suitable carriers include, but are not limited to, dimethylsulfoxide, ethanol, dicloromethane, methanol and the like.

Also described are methods for using the pharmaceutical composition described above. Described is administering an effective amount of one or more PTX-2 fusion proteins having one or more protomers having an attached antibody or antibody fragment. The antibodies or antibody fragments may be fused to the C- or N-terminal or inserted internally into the primary sequence of PTX-2 protomer, or otherwise attached to the PTX-2 protomer. The PTX-2 fusion protein may include two or more different antibodies or antibody fragments.

The disease state treated using such methods may vary and may vary depending upon the attached antibody or antibody fragment, or combination of antibodies or antibody fragments. However, the diseased state may be inflammation or chronic inflammation, such as inflammation associated with arthritis or rheumatoid arthritis, and in such embodiments, treatment may rely at least in part on the natural propensity of the PTX-2 fusion protein to accumulate in areas of inflammation.

### EXAMPLES

Although the present disclosure has been described in considerable detail with reference to certain preferred embodiments thereof, other versions are possible. Various aspects of the present disclosure will be illustrated with reference to the following non-limiting examples.

### EXAMPLE 1

### Preparation of anti-TNFα fusion protein

An expression vector was designed to encode a fusion protein of including an anti-TNFα antibody fragment (VHH3), a linker, and a PTX-2 primary sequence. The fusion protein (anti-TNFα-VHH3-PTX-2) produced from this expression vector was expected to be 364aa with a molecular weight: 40451.5 (aglycosylated) and was expected to include an N-terminal anti-TNFα antibody fragment that was linked to the PTX-2 protomer through a linker. This expression vector was transformed into a cell line based on the GPEx® technology (Catalent Middleton, Middleton, WI).

The fusion protein was produced in a 1L fed-batch shake flask and clarified by centrifugation followed by 0.2 µm filtration. The filtrate was then loaded onto phosphoethanolamine affinity resin (PE) equilibrated in 50 mM HEPES/100 mM NaCl/1mM CaCl2, pH 8.0 to capture the fusion protein, and the column was washed with an equilibration buffer. The fusion protein was eluted with 50mM HEPES/100mM NaCl/5 mM EDTA, pH 8.0 followed by viral filtration through a Pall DV50 filter and then 0.2 µm filtration.

The eluted fusion protein was further purified over a Source 30Q anion exchange column (S30Q). The column was equilibrated in 50mM HEPES/10mM NaCl, pH 8.0, and the eluted protein was diluted 3-fold in equilibration buffer before being loaded onto the column. The fusion protein was then eluted with a 20-CV gradient to 250mM NaCl in 50mM HEPES, pH 8.0. Fractions from the elution peak were pooled and concentrated to 5 mg/mL using a Millipore 10kDa spin concentrator. Samples of the 5 mg/mL fusion protein were tested by *in vitro* bioassay and pharmacokinetic (PK) analyses (iv injection) in rats as well as SDS-PAGE, and SE-HPLC analysis.

SE-HPLC analysis was performed on all samples using an Agilent 1200 system at concentrations ranging from 0.5 to 115 mg/mL to assess aggregate content. The purified fusion protein was subjected to 5 freeze/thaw cycles (-20 to +20 °C), and approximately 10 µg of a sample after each cycle was loaded onto a Tosoh 3000 SW XL size-exclusion column with a column temperature of 25 °C. The mobile phase of 50 mM Tris/50 mM Sodium phosphate/0.25 M Sodium chloride pH 7.5 was run at a flow rate at 1.0 mL/min. Detection of the fusion protein was performed at 280 nm. The chromatogram resulting from these experiments are shown in **FIG. 6** as an overlay of the purified fusion protein before and after each of 5 freeze/thaw cycles, and the percent pentamer determined for each HPLC trace is provided in Table 1 below. These data indicated that little if any pentameric PTX-2 dissociates or aggregates during freeze thaw cycles and demonstrate the stability of the fusion protein to freeze/thaw cycling.

**Table 1**

| **Cycle** | **% Pentamer** |
|---|---|
| 0 | 93.5% |
| 1 | 93.6% |
| 2 | 94.2% |
| 3 | 93.4% |
| 4 | 93.3% |
| 5 | 93.2% |

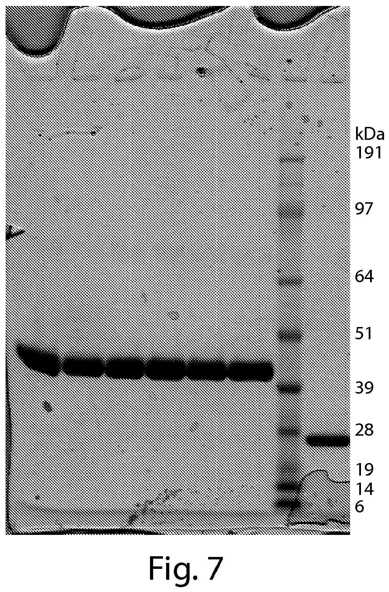

As in the SE-HPLC analysis described above, the purified anti-TNFa-VHH3-PTX-2 fusion protein was subjected to 5 freeze/thaw cycles (-20 to +20 °C), and approximately 4 µg of the protein per lane was loaded onto a 4-12% Bis-Tris SDS-PAGE gel using a MOPS running buffer (Invitrogen, Inc.). The gel was run at 150V for 50 minutes and stained using SimplyBlue Safe stain (Invitrogen, Inc.). An example of the resulting gel is provided in **FIG. 7** with lanes designated as follows:

| Lane | Sample |
|---|---|
| 1 | anti-TNFα-VHH3-PTX-2 0 F/T, 4 µg non-reduced |
| 2 | anti-TNFα-VHH3-PTX-2 1 F/T, 4 µg non-reduced |
| 3 | anti-TNFα-VHH3-PTX-2 2 F/T, 4 µg non-reduced |
| 4 | anti-TNFα-VHH3-PTX-2 3 F/T, 4 µg non-reduced |
| 5 | anti-TNFα-VHH3-PTX-2 4F /T, 4 µg non-reduced |
| 6 | anti-TNFα-VHH3-PTX-2 5 F/T, 4 µg non-reduced |
| 7 | SeeBlue+2 MWM |
| 8 | hPTX-2 std, 2.5 µg reduced |

These data show a fusion protein having the expected molecular weight (42 kDa). Additionally, the integrity of the 42 kDa band and absence of visible degradation products indicates that little or no degradation of the fusion protein results even after five freeze/thaw cycles.

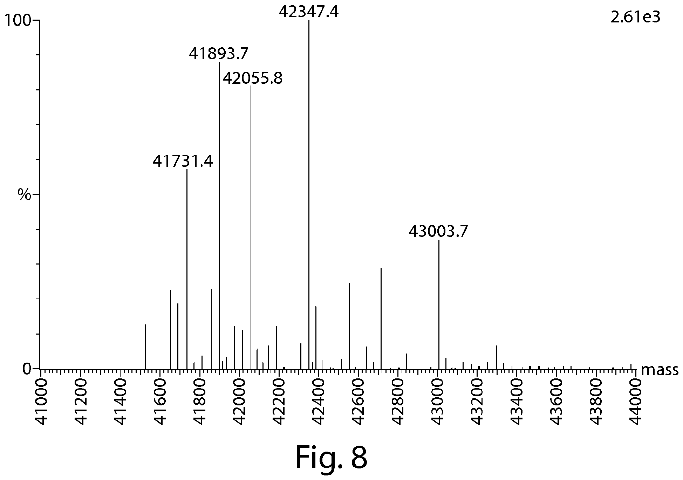

LC-MS analysis was performed on all samples to assess sialic acid content of glycans associated with the fusion protein. HPLC analysis was performed on a Waters 2695 interfaced to a Waters LCT Premier XE. Approximately 10 µg of each variant sample was injected onto a PerSeptive Biosystems Poros R2/10 column. Mobile phase A (0.05% formic acid in water) and mobile phase B (0.05% formic acid in acetonitrile) were run at 0.2 mL/min with a gradient of 30% B to 70% B over 5 minutes. The total effluent was injected into the mass spectrometer. The mass spectrometer was run with a capillary voltage of 3000, cone voltage of 50, desolvation temperature of 250 °C, source temperature of 120 °C, and cone gas and desolvation gas of 50 and 550 L/min, respectively. The MCP detector was set at a voltage of 2000. Data was analyzed with Mass Lynx 4.0 using MaxEnt, baseline subtraction, smoothing, and centering. All default parameters for each function were used. A representative trace is provided in **FIG. 8**. All labeled masses are expected glycoform variants of anti-TNFα-VHH3-PTX-2. These data show two major observed species masses of 42055 Da and 42347 Da, which correspond to the expected mass of the aglyco protein (40452 Da) plus the expected biantennary glycan structure (GlcNAc₂-Man₃-(GlcNAc-Gal)₂) with 0 and 1 terminal sialic acids, respectively.

### EXAMPLE 2

### Inhibition of monocyte differentiation

Peripheral blood mononuclear cells (PBMCs) were stimulated with M-CSF (Macrophage Colony Stimulating Factor) to promote differentiation of monocytes to fibrocytes and elevate the production of macrophage derived chemokine (MDC). PBMC's were obtained from Biological Specialty Corporation and plated at 50,000 cells per well in Supplemented FibroLife media (Life Line Cell Technology). The cells were treated with 25 ng/ml M-CSF to stimulate differentiation of monocytes into fibrocytes thereby increasing MDC release. Native PTX-2 (rhPTX-2) and anti-TNFα-VHH3-PTX-2, were serially diluted to achieve final concentrations in the wells ranging from 30 µg/ml to 0.0045 µg/ml. The cells were incubated for 96 hours at 37°C, 5% CO₂. Supernatants were extracted from the plates and tested in an MDC ELISA (R&D Systems), and MDC levels were measured to determine if the proteins dose-dependently inhibited MDC expression. The plates were read on a Tecan Infinite M200 plate reader using Magellan software. Percent inhibition was calculated and analyzed using Prism 4-parameter logistic fit analysis.

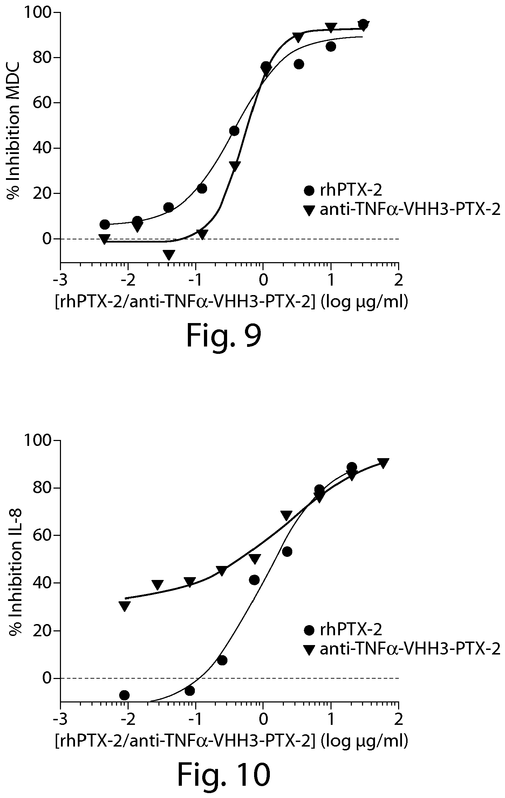

Exemplary curves showing the percent inhibition of MDC (% Inhibition MDC) for both rhPTX-2 and anti-TNFa-VHH3-PTX-2 are shown in **FIG. 9**. These data demonstrate similar dose dependent inhibition of monocyte to fibrocyte differentiation and MDC production, indicating that the fusion protein retains PTX-2 bioactivity.

### EXAMPLE 3

### Inhibition of IL-8 Activity

Immortalized monocyte/macrophage cells isolated from human peripheral blood (SC macrophage cells), were stimulated with Phorbol 12-myristate 13-acetate (PMA, Sigma-Aldrich) which increases IL-8 production. The cells were plated at 50,000 cells per well in Iscove's Modified Dulbecco's Medium containing 30 ng/ml PMA. rhPTX-2 and anti-TNFa-VHH3-PTX-2 were serially diluted to achieve final concentrations in the well ranging from 60 ug/ml to 0.009 ug/ml. The cells were incubated for 48 hours at 37°C, 5% CO₂. Supernatants were extracted from the plates and tested in an IL-8 ELISA (R&D Systems) following this incubation, and IL-8 levels were measured to determine if the proteins dose dependently inhibited IL-8 expression. The plates were read on a Tecan Infinite M200 plate reader using Magellan software. Percent inhibitions were calculated and analyzed using Prism 4-parameter logistic fit analysis.

Exemplary curves showing the percent IL-8 inhibition (% Inhibition IL-8) for both rhPTX-2 and anti-TNFa-VHH3-PTX-2 are shown in **FIG. 10**. These data show that both rhPTX-2 and anti-TNFa-VHH3-PTX-2 demonstrate dose dependent inhibition of IL-8. In addition, anti-TNFa-VHH3-PTX-2 appears more potent than rhPTX-2 in this assay, indicating unexpected synergy between PTX-2 bioactivity and anti-TNFa bioactivity when both activities are present on the same molecule.

### EXAMPLE 4

### Anti-TNFa activity

L929 cells were treated with cytotoxic TNFα in the presence of either rhPTX-2, Remicade, or anti-TNFα-VHH3-PTX-2 to evaluate the protective effects of anti-TNFα activity of a naked anti-TNFa antibody (Remicade®) in comparison to an anti-TNFα antibody-like fragment associated with PTX-2. rhPTX-2, Remicade, and anti-TNFα-VHH3-PTX-2 were individually serially diluted in assay media (Eagle's MEM containing 10% FBS and 2mM L-glutamine), and 25µl of human TNFα (1.5 ng/ml), 25 µl actinomycin D (40 µg/ml) and 50 µl of L929 cells (25,000 cells per well) were added to each of these test wells. Control wells containing 25 µl of assay media, 25 µl of actinomycin D and 50 µl of L929 cells and human TNFα at concentrations of 3125 pg/ml, 100 pg/ml, 1 pg/ml, and no TNFα were also present on the plate. Plates were incubated for 20 hours at 37°C, 5% CO₂. Following incubation, 30 µl MTS reagent (Promega®) was added to each well, and the plates were incubated for an additional 2 hours. Absorbance was read at 490 nm and viability curves were calculated using Excel and GraphPad Prism sigmoidal dose response analysis.

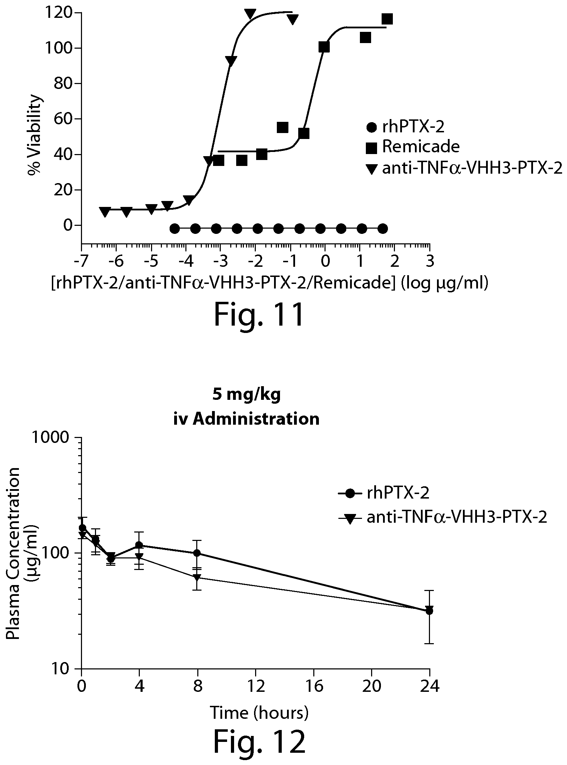

Exemplary results are provided in **FIG. 11**. As expected, rhPTX-2 demonstrates no activity in this assay, and anti-TNFα-VHH3-PTX-2 demonstrates potent protective effects against TNFα induced cytotoxicity, indicating that the fusion protein inhibits TNFα, an activity that was not present in native PTX-2. Moreover, these data show that anti-TNFα-VHH3-PTX-2 provides improved anti-TNFα inhibition activity when compared to Remicade, an industry standard for antibody based TNFα inhibition.

### EXAMPLE 5

### Pharmacokinetics

To show the pharmacokinetic activity of anti-TNFα-VHH3-PTX-2, rhPTX-2 and anti-TNFa-VHH3-PTX-2 were administered to rats intravenously (iv) at a dose of 5 mg/kg. Plasma samples were drawn at various time points, and these samples were analyzed in a PTX-2 ELISA. Male Sprague Dawley rats (175-200 grams) were purchased from Hilltop Lab Animals (Scottdale, PA) with surgically implanted dual jugular vein catheters (JVC). On the day of use, rats were weighed and the patency of the JVCs was established. Rats with patent JVCs were dosed in the left JVC with 1.67 mL/kg, for a final dose of 5 mg/kg rhPTX-2 and 8.2 mg/kg anti-TNFα-VHH3-PTX-2 (resulting in a 5 mg/kg dose of rhPTX-2). Following dosing, the catheter was flushed with 0.5 mL of saline and then tied-off.

A length of extension tubing was connected to the right JVC and passed through the top of a specially designed shoebox cage that allowed for unrestrained blood sample collection. Samples (0.5 mL) of whole blood were collected into EDTA-lined 1 cc syringes predose, 5 min and 1, 2, 4, 8, and 24 hr after dosing. The collection catheter was flushed with approximately 0.4 mL of heparinized saline and blood was transferred into tubes containing 25 µL of 16 mM disodium EDTA. Tubes were maintained on ice until centrifuged at 15,000 RPM for 10 minutes at 4°C. Plasma was collected and transferred to a cryotube and stored at -80°C until analysis. Following the 8 hour collection, the extension tubing was disconnected, the JVC was tied-off, and the animal was returned to its home cage. A terminal blood collection was collected at 24 hr by cardiac puncture following CO₂ inhalation.

The concentrations of rhPTX-2 in plasma were determined using an enzyme-linked immunoassay (ELISA) method. Briefly, 96 well microplates were coated with a mouse monoclonal anti-PTX-2 antibody to capture all rhPTX-2 or fusion protein in the sample. A polyclonal rabbit anti-PTX-2 antibody was used for detection followed by a goat anti-rabbit horseradish peroxidase conjugate system. Optical density of each well was determined at 450nm with a correction at 630nm. As appropriate, a rhPTX-2 or anti-TNFα-VHH3-PTX-2 standard curve was included on each plate with a calibration range of 0.125 to 20.0 ng/mL, and a lower limit of quantitation of 0.25 ng/mL. Accounting for the minimum required sample dilution of 200 fold, this translates to an LLOQ of 50.0 ng/mL. All sample results were interpolated from the standard curve and converted from ng/mL to µg/mL.

An exemplary clearance chart is provided in **FIG. 12**. These data show similar pharmacokinetics between the two proteins. Thus, anti-TNFα-VHH3-PTX-2 exhibits a drug like distribution and clearance that is nearly identical to that of native PTX-2.

## Claims

1. A fusion protein comprising a serum amyloid P (PTX-2) pentamer having at least one PTX-2 protomer fused with an antibody or antigen-binding antibody fragment; wherein the antibody or antibody fragment is fused to the N-terminus of at least one PTX-2 protomer; and wherein the fusion protein further comprises a linker between the antibody or antigen-binding antibody fragment and the at least one PTX-2 protomer.

2. The fusion protein of claim 1, wherein the PTX-2 pentamer comprises five protomers fused with antibodies or antigen-binding antibody fragments.

3. The fusion protein of claim 1, wherein the PTX-2 pentamer comprises one or more unmodified PTX-2 protomers.

4. The fusion protein of any one of claims 1 to 3, wherein the PTX-2 pentamer comprises one or more PTX-2 protomers that are modified to eliminate a ligand binding site on naturally occurring PTX-2 protomers, a Ca²⁺ binding site on naturally occurring PTX-2 protomers, or a combination thereof.

5. The fusion protein of any one of claims 1 to 4, wherein the PTX-2 pentamer comprises one or more PTX-2 protomers having at least one additional cysteine amino acid that is not present in naturally occurring PTX-2 protomers.

6. The fusion protein of claim 5, wherein at least one cysteine amino acid participates in a disulfide bond with the antibody or antigen-binding antibody fragment.

7. The fusion protein of claim 5, wherein at least one cysteine amino acid participates in a disulfide bond with another cysteine of the PTX-2 protomer.

8. The fusion protein of any one of claims 1 to 7, wherein the antibody or antigen-binding antibody fragment is a known therapeutic agent.

9. The fusion protein of any one of claims 1 to 8, wherein the antigen-binding antibody fragment is selected from single chain variable fragments (scFv), camelid VHH proteins, and adnexins.

10. The fusion protein of any one of claims 1 to 9, wherein the antibody or antigen-binding antibody fragment is an anti-tissue inhibitor of matrix metalloproteinase (TIMP) antibody or antigen-binding antibody fragment or an anti-tumor necrosis factor (TNF) antibody or antigen-binding antibody fragment.

11. The fusion protein of any one of claims 1 to 10, wherein the antibody or antigen-binding antibody fragment are anti-inflammatory.

12. A pharmaceutical composition comprising the fusion protein of any one of claims 1 to 11 and one or more pharmaceutically acceptable carriers or excipients.

13. A method for preparing a functionalized PTX-2 pentamer comprising:
introducing a first plasmid at least including a nucleotide sequence for a first functionalized PTX-2 protomer into an expression system, wherein the first plasmid at least comprises a nucleotide sequence that encodes an amino acid sequence that is at least 95% identical to SEQ ID NO: 1 and a nucleotide sequence for one or more first antibodies or antigen-binding antibody fragments, wherein said functionalized PTX-2 protomer is a fusion protein as defined in any one of claims 1 to 11;
expressing the first functionalized PTX-2 protomer; and
isolating functionalized PTX-2 pentamers, purifying functionalized PTX-2 pentamers, or combinations thereof from the expression system.

14. A PTX-2 pentamer for use in a method for treating a patient in need of treatment, wherein the PTX-2 pentamer is a fusion protein according to any one of claims 1 to 11.

15. The pentamer for use according to claim 14, wherein the antigen-binding antibody fragment is a single chain variable fragment (scFv), camelid VHH protein, or adnexin.

16. The pentamer for use according to claim 14, wherein the antibody or antigen-binding antibody fragment is an anti-tissue inhibitor of matrix metalloproteinase (TIMP) antibody or antigen-binding antibody fragment or an antitumor necrosis factor (TNF) antibody or antigen-binding antibody fragment.

17. The pentamer for use according to claim 14, wherein the pentamer is to be administered to reduce inflammation.

18. A method for preparing a colloid of PTX-2 comprising:
isolating PTX-2 pentamers, purifying PTX-2 pentamers, or combinations thereof; and adding calcium to the isolated and/or purified PTX-2 pentamers, wherein the PTX-2 pentamers are fusion proteins according to any one of claims 1 to 11.

## Patentansprüche

1. Fusionsprotein umfassend ein Serum-Amyloid-P (PTX-2) Pentamer, das mindestens ein PTX-2 Protomer hat, verbunden mit einem Antikörper oder Antigen-bindendem Antikörper-Fragment;
wobei der Antikörper oder das Antikörper-Fragment mit dem N-Terminus von mindestens einem PTX-2 Protomer verbunden ist, und wobei das Fusionsprotein des weiteren einen Linker zwischen dem Antikörper oder Antigen-bindendem Antikörper-Fragment und dem mindestens einem PTX-2 Protomer umfasst.

2. Fusionsprotein nach Anspruch 1, wobei das PTX-2 Pentamer fünf Protomere umfasst, die mit Antikörpern oder Antigen-bindenden Antikörper-Fragmenten verbunden sind.

3. Fusionsprotein nach Anspruch 1, wobei das PTX-2 Pentamer ein oder mehrere nicht-modifizierte PTX-2 Protomere umfasst.

4. Fusionsprotein nach einem der Ansprüche 1 bis 3, wobei das PTX-2 Pentamer ein oder mehrere PTX-2 Protomere umfasst, die modifiziert sind, so dass eine Liganden-Bindestelle auf natürlich vorkommenden PTX-2 Protomeren, eine Ca²⁺-Bindestelle auf natürlich vorkommenden PTX-2 Protomeren, oder eine Kombination davon, entfernt wurde.

5. Fusionsprotein nach einem der Ansprüche 1 bis 4, wobei das PTX-2 Pentamer ein oder mehrere PTX-2 Protomere umfasst, das/die mindestens eine zusätzliche Cystein-Aminosäure hat/haben, die nicht in natürlich vorkommenden PTX-2 Protomeren vorhanden ist.

6. Fusionsprotein nach Anspruch 5, wobei mindestens eine Cystein-Aminosäure an einer Disulfid-Bindung mit dem Antikörper oder dem Antigen-bindenden Antikörper-Fragment beteiligt ist.

7. Fusionsprotein nach Anspruch 5, wobei mindestens eine Cystein-Aminosäure an einer Disulfid-Bindung mit einem anderen Cystein des PTX-2 Protomers beteiligt ist.

8. Fusionsprotein nach einem der Ansprüche 1 bis 7, wobei der Antikörper oder das Antigen-bindende Antikörper-Fragment ein bekanntes therapeutisches Agens ist.

9. Fusionsprotein nach einem der Ansprüche 1 bis 8, wobei das Antigen-bindende Antikörper-Fragment ausgewählt ist aus Einzelketten-variablen-Fragmenten ("single chain variable fragments", scFv), Kamel-VHH-Proteinen und Adnexinen.

10. Fusionsprotein nach einem der Ansprüche 1 bis 9, wobei der Antikörper oder das Antigen-bindende Antikörper-Fragment ein anti-Gewebe-Inhibitor ("anti-tissue inhibitor") der Matrix-Metalloproteinase (TIMP) Antikörper oder Antigen-bindendes Antikörper-Fragment, oder ein anti-Tumor-Nekrose-Faktor (TNF) Antikörper oder Antigen-bindendes Antikörper-Fragment ist.

11. Fusionsprotein nach einem der Ansprüche 1 bis 10, wobei der Antikörper oder das Antigen-bindende Antikörper-Fragment entzündungshemmend sind.

12. Pharmazeutische Zusammensetzung, umfassend das Fusionsprotein nach einem der Ansprüche 1 bis 11 und einen oder mehrere pharmazeutisch verträgliche Träger oder Hilfsstoffe.

13. Verfahren zum Herstellen eines funktionalisierten PTX-2 Pentamers, umfassend:
Einführen eines ersten Plasmids, das mindestens eine Nukleotidsequenz für ein erstes, funktionalisiertes PTX-2 Protomer enthält, in ein Expressionssystem, wobei das erste Plasmid mindestens eine Nukleotidsequenz umfasst, die eine Aminosäuresequenz kodiert, die mindestens 95% identisch zu SEQ ID NO:1 ist, und eine Nukleotidsequenz umfasst für einen oder mehrere erste Antikörper oder Antigen-bindende Antikörper-Fragmente, wobei das funktionalisierte PTX-2 Protomer ein Fusionsprotein wie in einem der Ansprüche 1 bis 11 definiert ist;
Exprimieren des ersten, funktionalisierten PTX-2 Protomers; und
Isolieren von funktionalisierten PTX-2 Pentameren aus dem Expressionssystem, Aufreinigen von funktionalisierten PTX-2 Pentameren aus dem Expressionssystem, oder Kombinationen davon.

14. PTX-2 Pentamer zur Verwendung in einem Verfahren zur Behandlung eines Patienten, der Behandlung benötigt, wobei das PTX-2 Pentamer ein Fusionsprotein gemäß einem der Ansprüche 1 bis 11 ist.

15. Pentamer zur Verwendung gemäß Anspruch 14, wobei das Antigen-bindende Antikörper-Fragment ein Einzelketten-variables-Fragment ("single chain variable fragment", scFv), Kamel-VHH-Protein oder Adnexin ist.

16. Pentamer zur Verwendung gemäß Anspruch 14, wobei der Antikörper oder das Antigen-bindende Antikörper-Fragment ein anti-Gewebe-Inhibitor ("antitissue inhibitor") der Matrix-Metalloproteinase (TIMP) Antikörper oder Antigen-bindendes Antikörper-Fragment, oder ein anti-Tumor-Nekrose-Faktor (TNF) Antikörper oder Antigen-bindendes Antikörper-Fragment ist.

17. Pentamer zur Verwendung gemäß Anspruch 14, wobei das Pentamer zur Entzündungsverringerung zu verabreichen ist.

18. Verfahren zur Herstellung eines Kolloids von PTX-2 umfassend:
Isolieren von PTX-2 Pentameren, Aufreinigen von PTX-2 Pentameren, oder Kombinationen davon; und Hinzufügen von Kalzium zu den isolierten und/oder aufgereinigten PTX-2 Pentameren, wobei die PTX-2 Pentamere Fusionsproteine gemäß einem der Ansprüche 1 bis 11 sind.

## Revendications

1. Protéine de fusion comprenant un pentamère d'amyloïde sérique P (PTX-2) ayant au moins un protomère de PTX-2 fusionné à un anticorps ou à un fragment d'anticorps se liant à un antigène ;
dans laquelle l'anticorps ou le fragment d'anticorps est fusionné à l'extrémité N terminale d'au moins un protomère de PTX-2; et dans laquelle la protéine de fusion comprend en outre un lieur entre l'antigène ou le fragment d'anticorps se liant à un antigène et l'au moins un protomère de PTX-2.

2. Protéine de fusion selon la revendication 1, dans laquelle le pentamère de PTX-2 comprend cinq protomères fusionnés à des anticorps ou à des fragments d'anticorps se liant à un antigène.

3. Protéine de fusion selon la revendication 1, dans laquelle le pentamère de PTX-2 comprend un ou plusieurs protomères de PTX-2 non modifiés.

4. Protéine de fusion selon l'une quelconque des revendications 1 à 3, dans laquelle le pentamère de PTX-2 comprend un ou plusieurs protomères de PTX-2 qui sont modifiés pour éliminer un site de liaison à un ligand sur des protomères de PTX-2 naturels, un site de liaison à Ca²⁺ sur des protomères de PTX-2 naturels, ou une combinaison de ceux-ci.

5. Protéine de fusion selon l'une quelconque des revendications 1 à 4, dans laquelle le pentamère de PTX-2 comprend un ou plusieurs protomères de PTX-2 ayant au moins un acide aminé cystéine additionnel qui n'est pas présent dans les protomères de PTX-2 naturels.

6. Protéine de fusion selon la revendication 5, dans laquelle au moins un acide aminé cystéine participe à un pont disulfure avec l'anticorps ou le fragment d'anticorps se liant à un antigène.

7. Protéine de fusion selon la revendication 5, dans laquelle au moins un acide aminé cystéine participe à un pont disulfure avec une autre cystéine du protomère de PTX-2.

8. Protéine de fusion selon l'une quelconque des revendications 1 à 7, dans laquelle l'anticorps ou le fragment d'anticorps se liant à un antigène est un agent thérapeutique connu.

9. Protéine de fusion selon l'une quelconque des revendications 1 à 8, dans laquelle le fragment d'anticorps se liant à un antigène est choisi parmi les fragments variables à simple chaîne (scFv), les protéines VHH de camélidés, et les adnexines.

10. Protéine de fusion selon l'une quelconque des revendications 1 à 9, dans laquelle l'anticorps ou le fragment d'anticorps se liant à un antigène est un anticorps ou un fragment d'anticorps se liant à un antigène anti-inhibiteur tissulaire de métalloprotéinase de matrice (TIMP) ou un anticorps ou un fragment d'anticorps se liant à un antigène anti-facteur nécrosant les tumeurs (TNF).

11. Protéine de fusion selon l'une quelconque des revendications 1 à 10, dans laquelle l'anticorps ou le fragment d'anticorps se liant à un antigène est anti-inflammatoire.

12. Composition pharmaceutique comprenant la protéine de fusion selon l'une quelconque des revendications 1 à 11 et un ou plusieurs véhicules ou excipients pharmaceutiquement acceptables.

13. Méthode de préparation d'un pentamère de PTX-2 fonctionnalisé comprenant :
l'introduction d'un premier plasmide incluant au moins une séquence nucléotidique pour un premier protomère de PTX-2 fonctionnalisé dans un système d'expression, le premier plasmide comprenant au moins une séquence nucléotidique qui code une séquence d'acides aminés qui est au moins 95 % identique à la SEQ ID NO : 1 et une séquence nucléotidique pour un ou plusieurs premiers anticorps ou fragments d'anticorps se liant à un antigène, ledit protomère de PTX-2 fonctionnalisé étant une protéine de fusion telle que définie dans l'une quelconque des revendications 1 à 11 ;
l'expression du premier protomère de PTX-2 fonctionnalisé ; et
l'isolation de pentamères de PTX-2 fonctionnalisés, la purification de pentamères de PTX-2 fonctionnalisés, ou des combinaisons de celles-ci à partir du système d'expression.

14. Pentamère de PTX-2 pour une utilisation dans une méthode de traitement d'un patient ayant besoin d'un traitement, le pentamère de PTX-2 étant une protéine de fusion selon l'une quelconque des revendications 1 à 11.

15. Pentamère pour une utilisation selon la revendication 14, dans lequel le fragment d'anticorps se liant à un antigène est un fragment variable à simple chaîne (scFv), une protéine VHH de camélidé, ou une adnexine.

16. Pentamère pour une utilisation selon la revendication 14, dans lequel l'anticorps ou le fragment d'anticorps se liant à un antigène est un anticorps ou un fragment d'anticorps se liant à un antigène anti-inhibiteur tissulaire de métalloprotéinase de matrice (TIMP) ou un anticorps ou un fragment d'anticorps se liant à un antigène anti-facteur nécrosant les tumeurs (TNF).

17. Pentamère pour une utilisation selon la revendication 14, lequel pentamère est destiné à être administré pour réduire une inflammation.

18. Méthode de préparation d'un colloïde de PTX-2, comprenant :
l'isolation de pentamères de PTX-2, la purification de pentamères de PTX-2, ou des combinaisons de celles-ci ; et l'addition de calcium aux pentamères de PTX-2 isolés et/ou purifiés,
dans laquelle les pentamères de PTX-2 sont des protéines de fusion selon l'une quelconque des revendications 1 à 11.
